# NEW EUROPEAN PATENT SPECIFICATION

(11) **EP 2 227 248 B2**
(45) Date of publication and mention of the opposition decision: **31.01.2018**
(45) Mention of the grant of the patent: 11.06.2014
(21) Application number: 08862064.6
(22) Date of filing: 26.11.2008
(51) Int. Cl.: A61K 39/00, C08B 37/00, C12P 19/04

(54) **ADJUVANTED GLUCANS**
ADJUVIERTE GLUKANE
GLUCANES AVEC ADJUVANT

(30) Priority: 26.11.2007 US 4396 P; 01.07.2008 US 133738 P
(43) Date of publication of application: 15.09.2010
(73) Proprietor: GlaxoSmithKline Biologicals SA, 1330 Rixensart (BE)
(72) Inventor: BERTI, Francesco, I-53100 Siena (IT); COSTANTINO, Paolo, I-53100 Siena (IT); ROMANO, Maria, Rosaria, I-53100 Siena (IT)
(74) Representative: Evans, Stephen John Eves
(86) International application number: PCT/IB2008/003582
(87) International publication number: WO 2009/077854

(56) References cited:
- EP-A- 0 640 348
- WO-A-03/097091
- WO-A-2009/068996
- WO-A1-90/14837
- WO-A2-03/097091
- WO-A2-2009/068996
- ANTONELLA TOROSANTUCCI ET AL.: "A novel glyco-conjugate vaccine against fungal pathogens" THE JOURNAL OF EXPERIMENTAL MEDICINE, vol. 202, no. 5, 5 September 2002 (2002-09-05), pages 597-606, XP002372227 cited in the application
- SELVARAJ ET AL: "Adjuvant and immunostimulatory effects of beta-glucan administration in combination with lipopolysaccharide enhances survival and some immune parameters in carp challenged with Aeromonas hydrophila" VETERINARY IMMUNOLOGY AND IMMUNOPATHOLOGY, AMSTERDAM, NL, vol. 114, no. 1-2, 15 November 2006 (2006-11-15), pages 15-24, XP005664768 ISSN: 0165-2427
- CASADEVALL ARTURO ET AL: "Polysaccharide-containing conjugate vaccines for fungal diseases" TRENDS IN MOLECULAR MEDICINE, vol. 12, no. 1, January 2006 (2006-01), pages 6-9, XP025229887 ISSN: 1471-4914
- JAMOIS FRANK ET AL: "Glucan-like synthetic oligosaccharides: iterative synthesis of linear oligo-beta-(1,3)-glucans and immunostimulatory effects", GLYCOBIOLOGY, vol. 15, no. 4, April 2005 (2005-04), pages 393-407, ISSN: 0959-6658
- TOROSANTUCCI A. ET AL: 'A novel glyco-conjugate vaccine against fungal pathogens' THE JOURNAL OF EXSPERIMENTAL MEDICINE vol. 202, no. 5, 05 September 2005, pages 597 - 606
- Prio US 61/004,396
- Prio US 61/133,738
- Prio US 61/004,333
- BROMURO C. ET AL: 'Beta-glucan-CRM 197 conjugates as candidates antifungal vaccines' VACCINE vol. 28, 2010, pages 2615 - 2623
- KELLY D.F. ET AL: 'Haemophilus influenzae type b conjugate vaccines' IMMUNOLOGY vol. 113, 2004, pages 163 - 174
- TOROSANTUCCI A. ET AL: 'Protection by anti-beta-glucan antibodies Is associated with restricted beta-1,3 glican binding specificity and inhibition of fungal growth and adherence' PLOS ONE vol. 4, 2009, pages 1 - 17
- ADAMO R. ET AL: 'Synthesis of laminarin frafments and evaluation of a beta-(1,3) glucan hexasaccaride-CRM 197 conjugate as vaccine candidate against Candida albicans' JOURNAL OF CARBOHYDRATE CHEMISTRY vol. 30, 2011, pages 249 - 280
- Laminarin information sheet from Sigma-Aldrich website
- READ S.M. ET AL: 'Analysis of the structural heterogeneity of laminarin b electrospray-ionisation-mass spectrometry' CARBOHYDRATE RESEARCH (ABSTRACT) vol. 281, no. 2, 1996, pages 187 - 201

## Description

This application claims the benefit of U.S. Provisional Application Serial No. 61/004,396, filed on 26th November 2007; and U.S. Provisional Application Serial No. 61/133,738, filed on 1st July 2008.

### TECHNICAL FIELD

The invention relates to vaccines, more particularly those against fungal infections and disease.

### BACKGROUND OF THE INVENTION

The use of β-glucans as anti-fungal vaccines is reviewed in reference 1.

Reference 2 reports the use of various β-glucans in immunisation studies, including laminarin, pustulan and 'GG-zym' (soluble *C.albicans* glucans obtained by digesting glucan ghosts with β-1,3-glucanase). The GG-zym and laminarin glucans were both conjugated to carrier proteins to improve their immunogenicity. Further information on the conjugated laminarin are reported in reference 3.

In reference 2 the GG-zym conjugate was administered with both complete and incomplete Freund's adjuvants. More generally, the β-glucan-containing compositions are disclosed as optionally containing adjuvants. In reference 3 the laminarin conjugate was administered with complete Freund's adjuvant or with cholera toxin adjuvant.

It is an object of the invention to provide further and better glucan-based compositions for inducing protective and/or therapeutic immune responses against infections.

### SUMMARY OF THE INVENTION

The present invention relates to immunogenic compositions comprising a glucan containing exclusively β-1,3-linkages, wherein said glucan is a single molecular species and is conjugated to a carrier protein and wherein the glucan comprises one or more sequences of at least five adjacent non-terminal residues linked to other residues only by β-1,3 linkages. The carrier protein in the immunogenic compositions of the invention is a bacterial toxin or toxoid, or a mutant thereof. In a particular embodiment, the carrier protein is CRM 197.

In certain embodiments, the glucan has a molecular weight of less than 100 kDa (e.g. less than 80, 70, 60, 50, 40, 30, 25, 20, or 15 kDa). In other embodiments, the glucan has 60 or fewer glucose monosaccharide units.

The glucan has exclusively β-1,3 linkages. In a particular embodiment, the glucan is a curdlan. The immunogenic compositions of the invention can include a pharmaceutically acceptable carrier.

The composition can comprise an adjuvant. The adjuvant can comprise one or more of an aluminium salt, such as an aluminium hydroxide; an oil-in-water emulsion; an immunostimulatory oligonucleotide; and/or an α-glycosylceramide. The adjuvant can comprise an outer membrane vesicle (OMV). The adjuvant can comprise an immunostimulatory oligonucleotide and a polycationic oligopeptide.

The present invention also relates to methods for raising an immune response in a mammal, comprising administering to the mammal a composition of the invention.

### BRIEF DESCRIPTION OF DRAWINGS

Figure 1 shows SDS-PAGE of saccharides and conjugates. Lanes are: (1) CRM197; (2) laminarin conjugated to CRM197; (3) hydrolysed curdlan conjugated to CRM197; (4) tetanus toxoid monomer, Tt; (5) laminarin conjugated to Tt; (6) hydrolysed curdlan conjugated to Tt.
Figure 2 shows SEC-HPLC profiles for conjugates. Figure 2A shows profiles for CRM197 conjugates, and Figure 2B shows profiles for Tt conjugates. The right-most peak in both cases is the profile of unconjugated carrier. The lowest peak is a curdlan conjugate. The third peak is a laminarin conjugate.
Figure 3 shows HPLC-SEC analysis of CRM197 pre- and post-conjugation to laminarin.
Figure 4 summarises the conjugation of synthetic glucans.
Figure 5 shows an SDS-PAGE analysis of conjugates of synthetic glucans.
Figure 6 shows SEC-HPLC profiles for laminarin conjugate lots 9 and 10.
Figure 7 shows IgG GMT against laminarin conjugates adjuvanted with, from left to right: (1) aluminium hydroxide; (2) aluminium hydroxide + CpG oligo; (3) MF59; (4) IC31, high dose (49.5µl of a sample having over 1000 nmol/ml oligodeoxynucleotide and 40 nmol/ml peptide); (5) IC31, low dose (90µl of a sample having over 100 nmol/ml oligodeoxynucleotide and 4 nmol/ml peptide); (6) α-galactosylceramide; or (7) α-galactosylceramide + aluminium hydroxide.
Figure 8 shows IgG GMT against laminarin conjugated to either CRM197 or tetanus toxoid combined with various individual and combined adjuvants administered by intrapertioneal administration.
Figure 9 shows IgG GMT against laminarin conjugated to either CRM197 or tetanus toxoid combined with various individual and combined adjuvants administered by subcutaneous administration.
Figure 10 shows IgG GMT against curdlan conjugated to either CRM197 or tetanus toxoid combined with various individual and combined adjuvants administered by intrapertioneal administration.
Figure 11 shows IgG GMT against curdlan conjugated to either CRM197 or tetanus toxoid combined with various individual and combined adjuvants administered by subcutaneous administration.
Figure 12 shows IgG GMT against laminarin conjugates at various saccharide doses.
Figure 13 shows IgG GMT against curdlan conjugates alone or combined with individual adjuvants at various saccharide doses.
Figure 14 shows IgG GMT (anti-GGZym and anti-laminarin) against laminarin conjugates alone or combined with individual adjuvants at various saccharide doses.
Figure 15 shows IgG GMT (anti-GGZym) against laminarin conjugates combined with various individual adjuvants administered by intrapertioneal, subcutaneous or intramuscular administration.
Figure 16 shows IgG GMT (anti-laminarin) against laminarin conjugates combined with various individual adjuvants administered by intrapertioneal, subcutaneous or intramuscular administration.
Figure 17 shows the accumulation of *C*. *albicans* in the kidneys of mice treated with the pre- and post-immunization sera from mice treated with laminarin conjugates combined with various individual adjuvants.
Figure 18 shows the accumulation of *C*. *albicans* in the kidneys of mice treated with the pre- and post-immunization sera from mice treated with laminarin conjugates combined with the MF59 adjuvant.
Figure 19 shows the UV spectrum of a commercially-available laminarin extracted from *Laminaria digitata* and the spectra of the same material after one, two or three steps of filtration using a depth filter.
Figure 20 shows the stability of liquid formulations of glucan conjugates at 37° C combined with various adjuvants.
Figure 21 shows the stability of liquid formulations of glucan conjugates at 2-8° C combined with various adjuvants.
Figure 22 shows the stability of lyophilised formulations of glucan conjugates at 4, 25 or 37°C.
Figure 23 shows IgG GMT (anti-laminarin) against synthetic glucan and laminarin conjugates alone or combined with various individual and combined adjuvants administered by intrapertioneal administration.
Figure 24 shows the survival rate of mice treated with laminarin conjugated to CRM197 combined with MF59 or CRM197 and MF59 alone prior to challenge with *C.albicans.*
Figure 25 shows the survival rate of mice treated with curdlan conjugated to CRM197 combined with MF59 or MF59 alone prior to challenge with *C.albicans.*
Figure 26 shows the survival rate of mice treated with two synthetic glucan conjugates combined with MF59 or MF59 alone prior to challenge with *C.albicans.*

### DETAILED DESCRIPTION OF THE INVENTION

The present invention provides an immunogenic composition comprising a glucan containing exclusively β-1,3-linkages, wherein said glucan is a single molecular species and is conjugated to a carrier protein, and wherein the glucan comprises one or more sequences of at least five adjacent non-terminal residues linked to other residues only by β-1,3 linkages wherein the carrier protein is a bacterial toxin or toxoid, or a mutant thereof. The inventors have found that glucans that are single molecular species may be more immunogenic than more polydisperse glucans, particularly when the composition also comprises an adjuvant. Preferably, the composition therefore comprises an adjuvant in addition to the glucan.

### The glucan

Glucans are glucose-containing polysaccharides found *inter alia* in fungal cell walls. The α-glucans include one or more α-linkages between glucose subunits, whereas β-glucans include one or more β-linkages between glucose subunits. The glucan used in accordance with the invention includes β-1,3-linkages.

The glucan is linear.

Full-length native β-glucans are insoluble and have a molecular weight in the megadalton range. It is preferred to use soluble glucans in immunogenic compositions of the invention. Solubilisation may be achieved by fragmenting long insoluble glucans. This may be achieved by hydrolysis or, more conveniently, by digestion with a glucanase (*e.g.* with a β-1,6-glucanase). As an alternative, short glucans can be prepared synthetically by joining monosaccharide building blocks.

Low molecular weight glucans are preferred, particularly those with a molecular weight of less than 100 kDa (*e.g.* less than 80, 70, 60, 50, 40, 30, 25, 20, or 15 kDa). It is also possible to use oligosaccharides *e.g.* containing 60 or fewer (*e.g.* 59, 58, 57, 56, 55, 54, 53, 52, 51, 50, 49, 48, 47, 46, 45, 44, 43, 42, 41, 40 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4) glucose monosaccharide units. Within this range, oligosaccharides with between 10 and 50 or between 20 and 40 monosaccharide units are preferred.

The glucan may be a fungal glucan. A 'fungal glucan' will generally be obtained from a fungus but, where a particular glucan structure is found in both fungi and non-fungi (*e.g.* in bacteria, lower plants or algae) then the non-fungal organism may be used as an alternative source. Thus the glucan may be derived from the cell wall of a *Candida,* such as *C.albicans,* or from *Coccidioides immitis, Trichophyton verrucosum, Blastomyces dermatidis, Cryptococcus neoformans, Histoplasma capsulatum, Saccharomyces cerevisiae, Paracoccidioides brasiliensis,* or *Pythiumn insidiosum.*

There are various sources of fungal β-glucans. For instance, pure β-glucans are commercially available *e.g.* pustulan (Calbiochem) is a β-1,6-glucan purified from *Umbilicaria papullosa.* β-glucans can be purified from fungal cell walls in various ways. Reference 7, for instance, discloses a two-step procedure for preparing a water-soluble β-glucan extract from *Candida,* free from cell-wall mannan, involving NaClO oxidation and DMSO extraction. The resulting product ('Candida soluble β-D-glucan' or 'CSBG') is mainly composed of a linear β-1,3-glucan with a linear β-1,6-glucan moiety. Similarly, reference 2 discloses the production of GG-zym from *Calbicans.* Such glucans from *C.albicans,* include (a) β-1,6-glucans with β-1,3-glucan lateral chains and an average degree of polymerisation of about 30, and (b) β-1,3-glucans with β-1,6-glucan lateral chains and an average degree of polymerisation of about 4.

The glucan is a β-1,3 glucan. Laminarins are found in brown algae and seaweeds. The β(1-3):β(1-6) ratios of laminarins vary between different sources *e.g.* it is as low as 3:2 in *Eisenia bicyclis* laminarin, but as high as 7:1 in *Laminaria digititata* laminarin [8]. Thus the glucan used with the invention may have a β(1-3):β(1-6) ratio of between 1.5:1 and 7.5:1 *e.g.* about 2:1, 3:1, 4:1, 5:1, 6:1 or 7:1. Optionally, the glucan may have a terminal mannitol subunit, *e.g.* a 1,1-α-linked mannitol residue [9]. The glucan may also comprise mannose subunits.

The glucan has exclusively β-1,3 linkages. The inventors have found that these glucans may be more immunogenic than glucans comprising other linkages, particularly glucans comprising β-1,3 linkages and a greater proportion of β-1,6 linkages. Thus the glucan is made solely of β-1,3-linked glucose residues (*e.g.* linear β-D-glucopyranoses with exclusively 1,3 linkages). Optionally, though, the glucan may include monosaccharide residues that are not β-1,3-linked glucose residues e.g. it may include β-1,6-linked glucose residues. The ratio of β-1,3-linked glucose residues to these other residues should be at least 8:1 (e.g. ≥9:1, ≥10:1, ≥11:1, ≥12:1, ≥13:1, ≥14:1, ≥15:1, ≥16:1, ≥17:1, ≥18:1, ≥19:1, ≥20:1, ≥25:1, ≥30:1, ≥35:1, ≥40:1, ≥45:1, ≥50:1, ≥75:1, ≥100:1, *etc.*). There are one or more (e.g. ≥1, ≥2, ≥3, ≥4, ≥5, ≥6, ≥7, ≥8, ≥9, ≥10, ≥11, ≥12, *etc.*) sequences of at least five (*e.g.* ≥5, ≥6, ≥7, ≥8, ≥9, ≥10, ≥11, ≥12, ≥13, ≥14, ≥15, ≥16, ≥17, ≥18, ≥19, ≥20, ≥30, ≥40, ≥50, ≥60, *etc.*) adjacent non-terminal residues linked to other residues only by β-1,3 linkages. By "non-terminal" it is meant that the residue is not present at a free end of the glucan. In some embodiments, the adjacent non-terminal residues may not include any residues coupled to a carrier molecule, linker or other spacer as described below. The inventors have found that the presence of five adjacent non-terminal residues linked to other residues only by β-1,3 linkages may provide a protective antibody response, *e.g.* against *C.albicans.*

Where a β-glucan includes both β-1,3 and β-1,6 linkages at a desired ratio and/or sequence then this glucan may be found in nature (e.g. a laminarin), or it may be made artificially. For instance, it may be made by chemical synthesis, in whole or in part. Methods for the chemical synthesis of β-1,3/β-1,6 glucans are well known in the art, for example from references 10-20. β-glucan including both β-1,3 and β-1,6 linkages at a desired ratio may also be made starting from an available glucan and treating it with a β-1,6-glucanase (also known as glucan endo-1,6-β-glucosidase, 1,6-β-D-glucan glucanohydrolase, *etc.*; EC 3.2.1.75) or a β-1,3-glucanase (such as an exo-1,3-glucanase (EC 3.2.1.58) or an endo-1,3-glucanase (EC 3.2.1.39) until a desired ratio and/or sequence is reached.

When a glucan containing solely of β-1,3-linked glucose is desired then β-1,6-glucanase treatment may be pursued to completion, as β-1,6-glucanase will eventually yield pure β-1,3 glucan. More conveniently, however, a pure β-1,3-glucan may be used. These may be made synthetically, by chemical and/or enzymatic synthesis e.g. using a (1→3)-β-D-glucan synthase, of which several are known from many organisms (including bacteria, yeasts, plants and fungi). Methods for the chemical synthesis of β-1,3 glucans are well known in the art, for example from references 21-24. As a useful alternative to synthesis, a natural β-1,3-glucan may be used, such as a curdlan (linear β-1,3-glucan from an *Agrobacterium* previously known as *Alcaligenes faecalis var. myxogenes*; commercially available *e.g.* from Sigma-Aldrich catalog C7821) or paramylon (β-1,3-glucan from *Euglena*). Organisms producing high levels of β-1,3-glucans are known in the art e.g. the *Agrobacterium* of refs. 25 & 26, or the *Euglena gracilis* of ref. 27.

Laminarin and curdlan are typically found in nature as high molecular weight polymers e.g. with a molecular weight of at least 100kDa. They are often insoluble in aqueous media. In their natural forms, therefore, they are not well suited to immunization. Thus the invention may use a shorter glucan e.g. those containing 60 or fewer glucose monosaccharide units (e.g. 59, 58, 57, 56, 55, 54, 53, 52, 51, 50, 49, 48, 47, 46, 45, 44,43,42, 41, 40 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4). A glucan having a number of glucose residues in the range of 2-60 may be used e.g. between 10-50 or between 20-40 glucose units. A glucan with 25-30 glucose residues is particularly useful. Suitable glucans may be formed e.g. by acid hydrolysis of a natural glucan, or by enzymatic digestion *e.g.* with a glucanase, such as a β-1,3-glucanase. A glucan with 11-19, e.g. 13-19 and particularly 15 or 17, glucose monosaccharide units is also useful. In particular, glucans with structure (A) are specifically envisaged for use in the present invention: 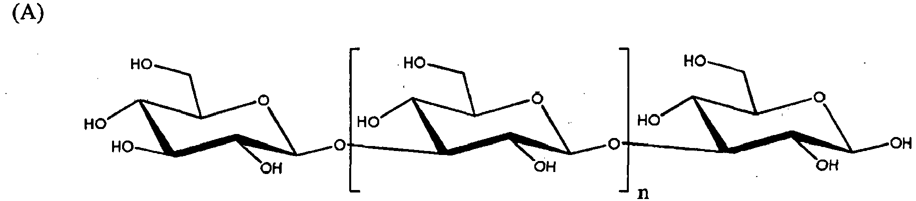 wherein n+2 is in the range of 2-60, e.g. between 10-50 or between 2-40. Preferably, n+2 is in the range of 25-30 or 11-19, *e.g.* 13-17. The inventors have found that n+2 = 15 is suitable.

The glucan (as defined above) is a single molecular species. In this embodiment, all of the glucan molecules are identical in terms of sequence. Accordingly, all of the glucan molecules are identical in terms of their structural properties, including molecular weight *etc.* Typically, this form of glucan is obtained by chemical synthesis, *e.g.* using the methods described above. For example, reference 22 describes the synthesis of a single β-1,3 linked species. Alternatively, in other embodiments, the glucan may be obtained from a natural glucan, *e.g.* a glucan from *L.digitata, Agrobacterium* or *Euglena* as described above, with the glucan being purified until the required single molecular species is obtained. Natural glucans that have been purified in this way are commercially available. A glucan that is a single molecular species may be identified by measuring the polydispersity (Mw/Mn) of the glucan sample. This parameter can conveniently be measured by SEC-MALLS, for example as described in reference 28. Suitable glucans for use in this embodiment of the invention have a polydispersity of about 1, *e.g.* 1.01 or less.

Solubility of natural glucans, such as curdlan, can be increased by introducing ionic groups (*e.g.* by sulfation, particularly at O-6 in curdlan). Such modifications may be used with the invention, but are ideally avoided as they may alter the glucan's antigenicity.

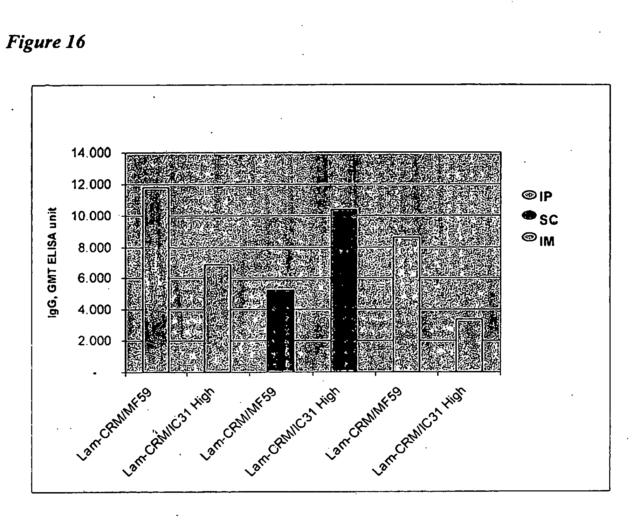

When glucans are isolated from natural sources, they may be isolated in combination with contaminants. For example, the inventors have found that glucans may be contaminated with phlorotannin, which is identifiable by ultraviolet-visible (UV/VIS) spectroscopy. This problem is particularly common when the glucan is isolated from a brown alga or seaweed. For example, the UV spectrum of a commercially-available laminarin extracted from *Laminaria digitata* includes an absorption peak resulting from the presence of phlorotannin contamination (Figure 16). Similarly, glucans extracted from *Artic laminarialis, Saccorhiza dermatodea* and *Alaria esculenta* have UV spectra that include an absorption peak resulting from phlorotannin contamination.

The presence of phlorotannin in a sample of glucan may affect the biological properties of the glucan. Accordingly, it may be desirable to remove phlorotannin from the sample, especially when the glucan is for medical or nutritional use.

Any suitable method of carrying out UVNIS spectroscopy may be used to measure the phlorotannin contamination of the glucan. For example, the inventors have found that suitable UV/VIS spectra may be obtained using a Perkin-Elmer LAMBDA™ 25 spectrophotometer at room temperature and pressure, using a quartz cell with a 1 cm path length containing glucan at 1mg/ml in water. The skilled person would be capable of selecting other suitable methods and conditions for obtaining UV/VIS spectra.

Other methods of measuring phlorotannin are known in the art. For example, high performance liquid chromatography was used for the detection and quantification of phlorotannins in reference 30. The skilled person would be capable of selecting methods that are suitable for measuring phlorotannin contamination in the present invention.

The phlorotannin may be separated from the glucan by any suitable method. For example, filtration methods may be used. The skilled person would be capable of selecting filters that have suitable properties for separating the phlorotannin from the glucan. Typically, the filter used to separate the phlorotannin from the glucan is a depth filter. Depth filters are well known to the person skilled in the art. Suitable depth filters include Cuno™ SP filters, which have a filter medium composed of an inorganic filter aid, cellulose and a resin that imparts a positive charge on the filter matrix. For example, the inventors have found that the Cuno™ 10 SP filter is particularly effective. However, other depth filters and other methods of filtration may also be used. For example, gel filtration may be used. Carbon-based filters may also be suitable. Carbon-based filters are well known to the person skilled in the art. They typically comprise a loose granular activated carbon bed or a pressed or extruded activated carbon block, which acts as a filter for purification of a sample. Alternatively, chromatographic procedures may be used to separate phlorotannin from the glucan. For example, affinity resin chromatography wherein the phlorotannin or the glucan is retained on the resin may be used.

The separation of phlorotannin from the glucan may be carried out in one (or 2, 3, 4, 5, 6, 7, 8, 9, 10 *etc.*) or more sub-steps. For example, the inventors have found that three sub-steps of filtration using a depth filter (*e.g.* a Cuno™ 10 SP filter) are particularly effective for reducing phlorotannin contamination.

The person skilled in the art is capable of identifying other separation techniques and conditions that result in the required reduction in phlorotannin contamination. For example, separation techniques and conditions may be optimised by carrying out a test separation step and then measuring residual phlorotannin contamination by the methods described above.

### Conjugates

Pure β-glucans are poor immunogens. For protective efficacy, therefore, β-glucans used with the invention are conjugated to a carrier protein. The use of conjugation to carrier proteins in order to enhance the immunogenicity of carbohydrate antigens is well known [e.g. reviewed in refs. 31 to 39 *etc.*] and is used in particular for paediatric vaccines [40].

The invention provides a composition comprising: (a) an immunogenic composition, as defined above and (b) an adjuvant, as defined above.

The carrier molecule may be covalently conjugated to the glucan directly or via a linker. Any suitable conjugation reaction can be used, with any suitable linker where necessary.

Attachment of the glucan antigen to the carrier is preferably via a -NH₂ group e.g. in the side chain of a lysine residue in a carrier protein, or of an arginine residue. Where a glucan has a free aldehyde group then this can react with an amine in the carrier to form a conjugate by reductive amination. Attachment to the carrier may also be via a -SH group e.g. in the side chain of a cysteine residue. Alternatively the glucan antigen may be attached to the carrier via a linker molecule.

The glucan will typically be activated or functionalised prior to conjugation. Activation may involve, for example, cyanylating reagents such as CDAP (e.g. 1-cyano-4-dimethylamino pyridinium tetrafluoroborate [41, 42, *etc*.]). Other suitable techniques use carbodiimides, hydrazides, active esters, norborane, p-nitrobenzoic acid, N-hydroxysuccinimide, S-NHS, EDC, TSTU (see also the introduction to reference 43).

Direct linkages to the protein may comprise oxidation of the glucan followed by reductive amination with the protein, as described in, for example, references 44 and 45.

Linkages via a linker group may be made using any known procedure, for example, the procedures described in references 46 and 47. Typically, the linker is attached via the anomeric carbon of the glucan. A preferred type of linkage is an adipic acid linker, which may be formed by coupling a free -NH₂ group (e.g. introduced to a glucan by amination) with adipic acid (using, for example, diimide activation), and then coupling a protein to the resulting saccharide-adipic acid intermediate [35, 48, 49]. A similar preferred type of linkage is a glutaric acid linker, which may be formed by coupling a free-NH₂ group with glutaric acid in the same way. Adipid and glutaric acid linkers may also be formed by direct coupling to the glucan, i.e. without prior introduction of a free group, e.g. a free -NH₂ group, to the glucan, followed by coupling a protein to the resulting saccharide-adipic/glutaric acid intermediate. Another preferred type of linkage is a carbonyl linker, which may be formed by reaction of a free hydroxyl group of a modified glucan with CDI [50, 51] followed by reaction with a protein to form a carbamate linkage. Other linkers include β-propionamido [52], nitrophenyl-ethylamine [53], haloacyl halides [54], glycosidic linkages [55], 6-aminocaproic acid [56], N-succinimidyl-3-(2-pyridyldithio)-propionate (SPDP) [57], adipic acid dihydrazide ADH [58], C₄ to C₁₂ moieties [59], *etc.* Carbodiimide condensation can also be used [60].

A bifunctional linker may be used to provide a first group for coupling to an amine group in the glucan (e.g. introduced to the glucan by amination) and a second group for coupling to the carrier (typically for coupling to an amine in the carrier). Alternatively, the first group is capable of direct coupling to the glucan, *i.e.* without prior introduction of a group, e.g. an amine group, to the glucan.

In some embodiments, the first group in the bifunctional linker is thus able to react with an amine group (-NH₂) on the glucan. This reaction will typically involve an electrophilic substitution of the amine's hydrogen. In other embodiments, the first group in the bifunctional linker is able to react directly with the glucan. In both sets of embodiments, the second group in the bifunctional linker is typically able to react with an amine group on the carrier. This reaction will again typically involve an electrophilic substitution of the amine.

Where the reactions with both the glucan and the carrier involve amines then it is preferred to use a bifunctional linker. For example, a homobifunctional linker of the formula X-L-X, may be used where: the two X groups are the same as each other and can react with the amines; and where L is a linking moiety in the linker. Similarly, a heterobifunctional linker of the formula X-L-X may be used, where: the two X groups are different and can react with the amines; and where L is a linking moiety in the linker. A preferred X group is N-oxysuccinimide. L preferably has formula L'-L²-L', where L' is carbonyl. Preferred L² groups are straight chain alkyls with 1 to 10 carbon atoms (e.g. C₁, C₂, C₃, C₄, C₅, C₆, C₇, C₈, C₉, C₁₀) *e.g.* -(CH₂)₄- or -(CH₂)₃.

Similarly, where the reaction with the glucan involves direct coupling and the reaction with the carrier involves an amine then it is also preferred to use a bifunctional linker. For example, a homobifunctional linker of the formula X-L-X may be used, where: the two X groups are the same as each other and can react with the glucan/amine; and where L is a linking moiety in the linker. Similarly, a heterobifunctional linker of the formula X-L-X may be used, where: the two X groups are different and one can react with the glucan while the other can react with the amine; and where L is a linking moiety in the linker. A preferred X group is N-oxysuccinimide. L preferably has formula L'-L²-L', where L' is carbonyl. Preferred L² groups are straight chain alkyls with 1 to 10 carbon atoms (e.g. C₁, C₂, C₃, C₄, C₅, C₆, C₇, C₈, C₉, C₁₀) e.g. -(CH₂)₄- or -(CH₂)₃-.

Other X groups for use in the bifunctional linkers described in the two preceding paragraphs are those which form esters when combined with HO-L-OH, such as norborane, p-nitrobenzoic acid, and sulfo-N-hydroxysuccinimide.

Further bifunctional linkers for use with the invention include acryloyl halides (e.g. chloride) and haloacylhalides.

The linker will generally be added in molar excess to glucan during coupling to the glucan.

The carrier proteins are bacterial toxins, such as diphtheria or tetanus toxins, or toxoids or mutants thereof. These are commonly used in conjugate vaccines. The CRM₁₉₇ diphtheria toxin mutant is particularly preferred [61].

Carrier proteins include the *N.meningitidis* outer membrane protein complex [62], synthetic peptides [63,64], heat shock proteins [65,66], pertussis proteins [67,68], cytokines [69], lymphokines [69], hormones [69], growth factors [69], artificial proteins comprising multiple human CD4⁺ T cell epitopes from various pathogen-derived antigens [70] such as N19 [71], protein D from *H.influenzae* [72-74], pneumolysin [75] or its non-toxic derivatives [76], pneumococcal surface protein PspA [77], iron-uptake proteins [78], toxin A or B from *C.difficile* [79], recombinant *Pseudomonas aeruginosa* exoprotein A (rEPA) [80], *etc.* It is possible to use mixtures of carrier proteins. A single carrier protein may carry multiple different glucans [81].

Conjugates may have excess carrier (w/w) or excess glucan (w/w) e.g. in the ratio range of 1:5 to 5:1. Conjugates with excess carrier protein are typical *e.g.* in the range 0.2:1 to 0.9:1, or equal weights. The conjugate may include small amounts of free (*i.e*. unconjugated) carrier. When a given carrier protein is present in both free and conjugated form in a composition of the invention, the unconjugated form is preferably no more than 5% of the total amount of the carrier protein in the composition as a whole, and more preferably present at less than 2% (by weight).

When the conjugate forms the glucan component in an immunogenic composition of the invention, the composition may also comprise free carrier protein as immunogen [82].

After conjugation, free and conjugated glucans can be separated. There are many suitable methods e.g. hydrophobic chromatography, tangential ultrafiltration, diafiltration, *etc.* [see also refs. 83, 84 *etc.*]. Tangential flow ultrafiltration is preferred.

The glucan moiety in the conjugate is preferably a low molecular weight glucan, as defined above. Oligosaccharides will typically be sized prior to conjugation.

The protein-glucan conjugate is preferably soluble in water and/or in a physiological buffer.

The inventors have found that immunogenicity may be improved if there is a spacer between the glucan and the carrier protein. In this context, a "spacer" is a moiety that is longer than a single covalent bond. This spacer may be a linker, as described above. Alternatively, it may be a moiety covalently bonded between the glucan and a linker. Typically, the moiety will be covalently bonded to the glucan prior to coupling to the linker or carrier. For example, the spacer may be moiety Y, wherein Y comprises a straight chain alkyl with 1 to 10 carbon atoms (e.g. C₁, C₂, C₃, C₄, C₅, C₆, C₇, C₈, C₉, C₁₀), typically 1 to 6 carbon atoms (*e.g.* C₁, C₂, C₃, C₄, C₅, C₆). The inventors have found that a straight chain alkyl with 6 carbon atoms (*i.e.* -(CH₂)₆) is particularly suitable, and may provide greater immunogenicity than shorter chains (*e.g.* -(CH₂)₂). Typically, Y is attached to the anomeric carbon of the glucan, usually via an -O-linkage. However, Y may be linked to other parts of the glucan and/or via other linkages. The other end of Y is bonded to the linker by any suitable linkage. Typically, Y terminates with an amine group to facilitate linkage to a bifunctional linker as described above. In these embodiments, Y is therefore bonded to the linker by an -NH- linkage. Accordingly, a conjugate with the following structure is specifically envisaged for use in the present invention: 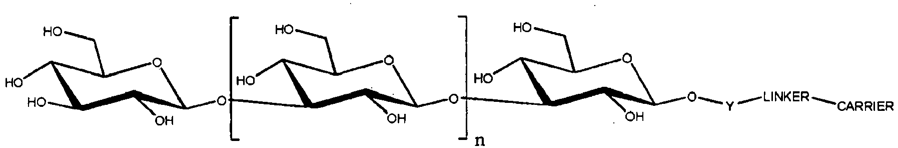 wherein n+2 is in the range of 2-60, e.g. between 10-50 or between 2-40. Preferably, n+2 is in the range of 25-30 or 11-19, *e.g.* 13-17. The inventors have found that n+2 = 15 is suitable. Y is as described above. "LINKER" is an optional linker as described above, while "CARRIER" is a carrier molecule as described above.

### Adjuvants

Even though β-glucans have themselves been reported to be adjuvants, an immunogenic composition may include a separate adjuvant, which can function to enhance the immune responses (humoral and/or cellular) elicited in a patient who receives the composition. Adjuvants that can be used with the invention include, but are not limited to:
- A mineral-containing composition, including calcium salts and aluminum salts (or mixtures thereof). Calcium salts include calcium phosphate (e.g. the "CAP" particles disclosed in ref. 85). Aluminum salts include hydroxides, phosphates, sulfates, *etc.*, with the salts taking any suitable form (*e.g.* gel, crystalline, amorphous, *etc.*). Adsorption to these salts is preferred. The mineral containing compositions may also be formulated as a particle of metal salt [86]. The adjuvants known as aluminum hydroxide and aluminum phosphate may be used. These names are conventional, but are used for convenience only, as neither is a precise description of the actual chemical compound which is present (*e.g.* see chapter 9 of reference 170). The invention can use any of the "hydroxide" or "phosphate" adjuvants that are in general use as adjuvants. The adjuvants known as "aluminium hydroxide" are typically aluminium oxyhydroxide salts, which are usually at least partially crystalline. The adjuvants known as "aluminium phosphate" are typically aluminium hydroxyphosphates, often also containing a small amount of sulfate (*i.e.* aluminium hydroxyphosphate sulfate). They may be obtained by precipitation, and the reaction conditions and concentrations during precipitation influence the degree of substitution of phosphate for hydroxyl in the salt. The invention can use a mixture of both an aluminium hydroxide and an aluminium phosphate. In this case there may be more aluminium phosphate than hydroxide *e.g.* a weight ratio of at least 2:1 *e.g.* ≥5:1, ≥6:1, ≥7:1, ≥8:1, ≥9:1, *etc.* The concentration of Al⁺⁺⁺ in a composition for administration to a patient is preferably less than 10mg/ml *e.g.* ≤5 mg/ml, ≤4 mg/ml, ≤3 mg/ml, ≤2 mg/ml, ≤1 mg/ml, *etc.* A preferred range is between 0.3 and 1mg/ml. A maximum of 0.85mg/dose is preferred.

- Saponins [chapter 22 of ref. 170], which are a heterologous group of sterol glycosides and triterpenoid glycosides that are found in the bark, leaves, stems, roots and even flowers of a wide range of plant species. Saponin from the bark of the *Quillaia saponaria* Molina tree have been widely studied as adjuvants. Saponin can also be commercially obtained from *Smilax ornata* (sarsaprilla), *Gypsophilla paniculata* (brides veil), and *Saponaria officianalis* (soap root). Saponin adjuvant formulations include purified formulations, such as QS21, as well as lipid formulations, such as ISCOMs. QS21 is marketed as Stimulon™. Saponin compositions have been purified using HPLC and RP-HPLC. Specific purified fractions using these techniques have been identified, including QS7, QS17, QS18, QS21, QH-A, QH-B and QH-C. Preferably, the saponin is QS21. A method of production of QS21 is disclosed in ref. 87. Saponin formulations may also comprise a sterol, such as cholesterol [88]. Combinations of saponins and cholesterols can be used to form unique particles called immunostimulating complexs (ISCOMs) [chapter 23 of ref. 170]. ISCOMs typically also include a phospholipid such as phosphatidylethanolamine or phosphatidylcholine. Any known saponin can be used in ISCOMs. Preferably, the ISCOM includes one or more of QuilA, QHA & QHC. ISCOMs are further described in refs. 88-90. Optionally, the ISCOMS may be devoid of additional detergent [91]. A review of the development of saponin based adjuvants can be found in refs. 92 & 93.
- Bacterial ADP-ribosylating toxins (*e.g.* the *E.coli* heat labile enterotoxin "LT", cholera toxin "CT", or pertussis toxin "PT"), and in particular detoxified derivatives thereof (*cf.* ref. 3), such as the mutant toxins known as LT-K63 and LT-R72 [94] or CT-E29H [95]. The use of detoxified ADP-ribosylating toxins as mucosal adjuvants is described in ref. 96 and as parenteral adjuvants in ref. 97.
- Bioadhesives and mucoadhesives, such as esterified hyaluronic acid microspheres [98] or chitosan and its derivatives [99].
- Microparticles (*i.e*. a particle of ∼100nm to ∼150µm in diameter, more preferably ∼200nm to ∼30µm in diameter, or ∼500nm to ∼10µm in diameter) formed from materials that are biodegradable and non-toxic (*e.g.* a poly(α-hydroxy acid), a polyhydroxybutyric acid, a polyorthoester, a polyanhydride, a polycaprolactone, *etc*.), with poly(lactide-co-glycolide) being preferred, optionally treated to have a negatively-charged surface (*e.g.* with SDS) or a positively-charged surface (*e.g.* with a cationic detergent, such as CTAB).
- Liposomes (Chapters 13 & 14 of ref. 170). Examples of liposome formulations suitable for use as adjuvants are described in refs. 100-102.
- Muramyl peptides, such as N-acetylmuramyl-L-threonyl-D-isoglutamine ("thr-MDP"), N-acetyl-normuramyl-L-alanyl-D-isoglutamine (nor-MDP), N-acetylglucsaminyl-N-acetylmuramyl-L-Al-D-isoglu-L-Ala-dipalmitoxy propylamide ("DTP-DPP", or "Theramide™), N-acetylmuramyl-L-alanyl-D-isoglutaminyl-L-alanine-2-(1'-2'dipalmitoyl-sn-glycero-3-hydroxyphosphoryloxy)-ethylamine ("MTP-PE").
- A polyoxidonium polymer [103,104] or other N-oxidized polyethylene-piperazine derivative.
- Methyl inosine 5'-monophosphate ("MIMP") [105].
- A polyhydroxlated pyrrolizidine compound [106], such as one having formula: 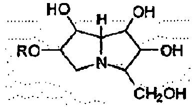 where R is selected from the group comprising hydrogen, straight or branched, unsubstituted or substituted, saturated or unsaturated acyl, alkyl (*e.g.* cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi*-casuarine, 7-*epi*-casuarine, 3,7-di*epi*-casuarine, *etc*.
- A CD1d ligand, such as an α-glycosylceramide [107-114] (e.g. α-galactosylceramide), phytosphingosine-containing α-glycosylceramides, OCH, KRN7000 [(2S,3S,4R)-1-O-(α-D-galactopyranosyl)-2-(N-hexacosanoylamino)-1,3,4-octadecanetriol], CRONY-101, 3"-O-sulfogalactosylceramide, *etc.*
- A gamma inulin [115] or derivative thereof, such as algammulin.
- An oil-in-water emulsion. Various such emulsions are known, and they typically include at least one oil and at least one surfactant, with the oil(s) and surfactant(s) being biodegradable (metabolisable) and biocompatible. The oil droplets in the emulsion are generally less than 5µm in diameter, and may even have a sub-micron diameter, with these small sizes being achieved with a microfluidiser to provide stable emulsions. Droplets with a size less than 220nm are preferred as they can be subjected to filter sterilization.
- An immunostimulatory oligonucleotide, such as one containing a CpG motif (a dinucleotide sequence containing an unmethylated cytosine residue linked by a phosphate bond to a guanosine residue), or a CpI motif (a dinucleotide sequence containing cytosine linked to inosine), or a double-stranded RNA, or an oligonucleotide containing a palindromic sequence, or an oligonucleotide containing a poly(dG) sequence. Immunostimulatory oligonucleotides can include nucleotide modifications/analogs such as phosphorothioate modifications and can be double-stranded or (except for RNA) single-stranded. References 116, 117 and 118 disclose possible analog substitutions e.g. replacement of guanosine with 2'-deoxy-7-deazaguanosine. The adjuvant effect of CpG oligonucleotides is further discussed in refs. 119-124. A CpG sequence may be directed to TLR9, such as the motif GTCGTT or TTCGTT [125]. The CpG sequence may be specific for inducing a Th1 immune response, such as a CpG-A ODN (oligodeoxynucleotide), or it may be more specific for inducing a B cell response, such a CpG-B ODN. CpG-A and CpG-B ODNs are discussed in refs. 126-128. Preferably, the CpG is a CpG-A ODN. Preferably, the CpG oligonucleotide is constructed so that the 5' end is accessible for receptor recognition. Optionally, two CpG oligonucleotide sequences may be attached at their 3' ends to form "immunomers". See, for example, references 125 & 129-131. A useful CpG adjuvant is CpG7909, also known as ProMune™ (Coley Pharmaceutical Group, Inc.). Another is CpG1826. As an alternative, or in addition, to using CpG sequences, TpG sequences can be used [132], and these oligonucleotides may be free from unmethylated CpG motifs. The immunostimulatory oligonucleotide may be pyrimidine-rich. For example, it may comprise more than one consecutive thymidine nucleotide (e.g. TTTT, as disclosed in ref. 132), and/or it may have a nucleotide composition with >25% thymidine (e.g. >35%, >40%, >50%, >60%, >80%, *etc.*). For example, it may comprise more than one consecutive cytosine nucleotide (e.g. CCCC, as disclosed in ref. 132), and/or it may have a nucleotide composition with >25% cytosine (e.g. >35%, >40%, >50%, >60%, >80%, *etc.*). These oligonucleotides maybe free from unmethylated CpG motifs. Immunostimulatory oligonucleotides will typically comprise at least 20 nucleotides. They may comprise fewer than 100 nucleotides.
   A particularly useful adjuvant based around immunostimulatory oligonucleotides is known as IC31™ [133]. Thus an adjuvant used with the invention may comprise a mixture of (i) an oligonucleotide (e.g. between 15-40 nucleotides) including at least one (and preferably multiple) CpI motifs, and (ii) a polycationic polymer, such as an oligopeptide (e.g. between 5-20 amino acids) including at least one (and preferably multiple) Lys-Arg-Lys tripeptide sequence(s). The oligonucleotide may be a deoxynucleotide comprising 26-mer sequence 5'-(IC)₁₃-3' (SEQ ID NO: 1). The polycationic polymer may be a peptide comprising 11-mer amino acid sequence KLKLLLLLKLK (SEQ ID NO: 2).
- 3-O-deacylated monophosphoryl lipid A ('3dMPL', also known as 'MPL™') [134-137]. In aqueous conditions, 3dMPL can form micellar aggregates or particles with different sizes e.g. with a diameter <150nm or >500nm. Either or both of these can be used with the invention, and the better particles can be selected by routine assay. Smaller particles (e.g. small enough to give a clear aqueous suspension of 3dMPL) are preferred for use according to the invention because of their superior activity [138]. Preferred particles have a mean diameter less than 220nm, more preferably less than 200nm or less than 150nm or less than 120nm, and can even have a mean diameter less than 100nm. In most cases, however, the mean diameter will not be lower than 50nm.
- An imidazoquinoline compound, such as Imiquimod ("R-837") [139,140], Resiquimod ("R-848") [141], and their analogs; and salts thereof (e.g. the hydrochloride salts). Further details about immunostimulatory imidazoquinolines can be found in references 142 to 146.
- A thiosemicarbazone compound, such as those disclosed in reference 147. Methods of formulating, manufacturing, and screening for active compounds are also described in reference 147. The thiosemicarbazones are particularly effective in the stimulation of human peripheral blood mononuclear cells for the production of cytokines, such as TNF-α.
- A tryptanthrin compound, such as those disclosed in reference 148. Methods of formulating, manufacturing, and screening for active compounds are also described in reference 148. The thiosemicarbazones are particularly effective in the stimulation of human peripheral blood mononuclear cells for the production of cytokines, such as TNF-α.
- A nucleoside analog, such as: (a) Isatorabine (ANA-245; 7-thia-8-oxoguanosine): 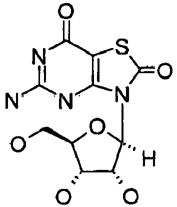 and prodrugs thereof; (b) ANA975; (c) ANA-025-1; (d) ANA380; (e) the compounds disclosed in references 149 to 151Loxoribine (7-allyl-8-oxoguanosine) [152].
- Compounds disclosed in reference 153, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [154,155], Hydrapthalamide compounds, Benzophenone compounds, Isoxazole compounds, Sterol compounds, Quinazilinone compounds, Pyrrole compounds [156], Anthraquinone compounds, Quinoxaline compounds, Triazine compounds, Pyrazalopyrimidine compounds, and Benzazole compounds [157].
- An aminoalkyl glucosaminide phosphate derivative, such as RC-529 [158,159].
- A phosphazene, such as poly[di(carboxylatophenoxy)phosphazene] ("PCPP") as described, for example, in references 160 and 161.
- A substituted urea or compound of formula I, II or III, or a salt thereof: 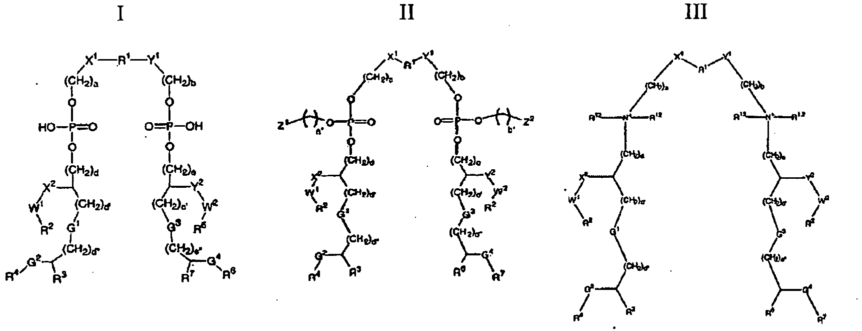 as defined in reference 162, such as 'ER 803058', 'ER 803732', 'ER 804053', ER 804058', 'ER 804059', 'ER 804442', 'ER 804680', 'ER 804764', ER 803022 or 'ER 804057' e.g.: 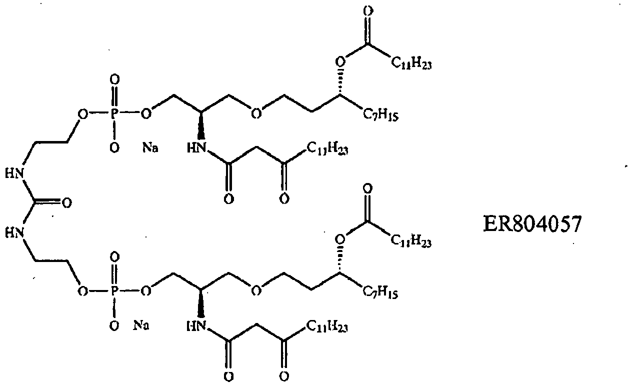 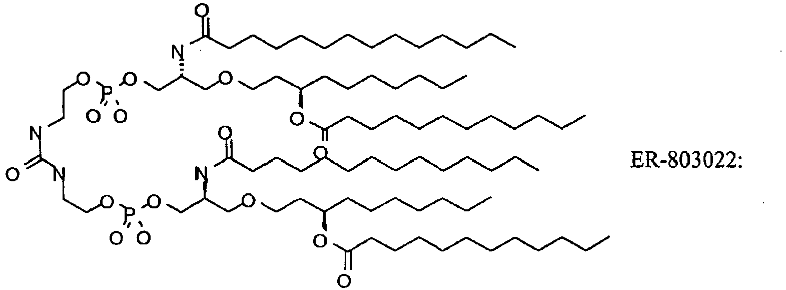
- Derivatives of lipid A from *Escherichia coli* such as OM-174 (described in refs. 163 & 164).
- Compounds containing lipids linked to a phosphate-containing acyclic backbone, such as the TLR4 antagonist E5564 [165,166]:

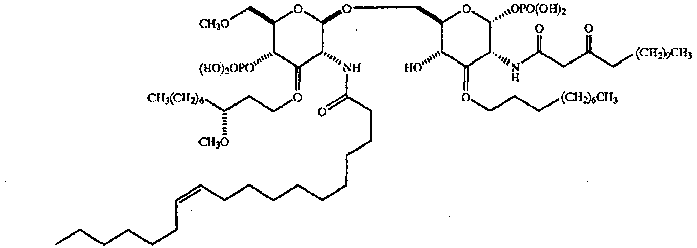

These and other adjuvant-active substances are discussed in more detail in references 170 & 171.

Specific oil-in-water emulsion adjuvants useful with the invention include, but are not limited to:
- A submicron emulsion of squalene, Tween 80, and Span 85. The composition of the emulsion by volume can be about 5% squalene, about 0.5% polysorbate 80 and about 0.5% Span 85. In weight terms, these ratios become 4.3% squalene, 0.5% polysorbate 80 and 0.48% Span 85. This adjuvant is known as 'MF59' [167-169], as described in more detail in Chapter 10 of ref. 170 and chapter 12 of ref. 171. The MF59 emulsion advantageously includes citrate ions e.g. 10mM sodium citrate buffer.
- An emulsion of squalene, a tocopherol, and Tween 80. The emulsion may include phosphate buffered saline. It may also include Span 85 (e.g. at 1%) and/or lecithin. These emulsions may have from 2 to 10% squalene, from 2 to 10% tocopherol and from 0.3 to 3% Tween 80, and the weight ratio of squalene:tocopherol is preferably ≤1 as this provides a more stable emulsion. Squalene and Tween 80 may be present volume ratio of about 5:2. One such emulsion can be made by dissolving Tween 80 in PBS to give a 2% solution, then mixing 90ml of this solution with a mixture of (5g of DL-α-tocopherol and 5ml squalene), then microfluidising the mixture. The resulting emulsion may have submicron oil droplets e.g. with an average diameter of between 100 and 250nm, preferably about 180nm.
- An emulsion of squalene, a tocopherol, and a Triton detergent (e.g. Triton X-100). The emulsion may also include a 3d-MPL (see below). The emulsion may contain a phosphate buffer.
- An emulsion comprising a polysorbate (e.g. polysorbate 80), a Triton detergent (e.g. Triton X-100) and a tocopherol (e.g. an α-tocopherol succinate). The emulsion may include these three components at a mass ratio of about 75:11:10 (e.g. 750µg/ml polysorbate 80, 110µg/ml Triton X-100 and 100µg/ml α-tocopherol succinate), and these concentrations should include any contribution of these components from antigens. The emulsion may also include squalene. The emulsion may also include a 3d-MPL (see below). The aqueous phase may contain a phosphate buffer.
- An emulsion of squalane, polysorbate 80 and poloxamer 401 ("Pluronic™ L121"). The emulsion can be formulated in phosphate buffered saline, pH 7.4. This emulsion is a useful delivery vehicle for muramyl dipeptides, and has been used with threonyl-MDP in the "SAF-1" adjuvant [172] (0.05-1% Thr-MDP, 5% squalane, 2.5% Pluronic L121 and 0.2% polysorbate 80). It can also be used without the Thr-MDP, as in the "AF" adjuvant [173] (5% squalane, 1.25% Pluronic L121 and 0.2% polysorbate 80). Microfluidisation is preferred.
- An emulsion having from 0.5-50% of an oil, 0.1-10% of a phospholipid, and 0.05-5% of a non-ionic surfactant. As described in reference 174, preferred phospholipid components are phosphatidylcholine, phosphatidylethanolamine, phosphatidylserine, phosphatidylinositol, phosphatidylglycerol, phosphatidic acid, sphingomyelin and cardiolipin. Submicron droplet sizes are advantageous.
- A submicron oil-in-water emulsion of a non-metabolisable oil (such as light mineral oil) and at least one surfactant (such as lecithin, Tween 80 or Span 80). Additives may be included, such as QuilA saponin, cholesterol, a saponin-lipophile conjugate (such as GPI-0100, described in reference 175, produced by addition of aliphatic amine to desacylsaponin via the carboxyl group of glucuronic acid), dimethyidioctadecylammonium bromide and/or N,N-dioctadecyl-N,N-bis (2-hydroxyethyl)propanediamine.
- An emulsion in which a saponin (e.g. QuilA or QS21) and a sterol (e.g. a cholesterol) are associated as helical micelles [176].

Preferred emulsion adjuvants have an average droplets size of <1µm e.g. ≤750nm, <500nm, ≤400nm, ≤300nm, ≤250nm, ≤220nm, ≤200nm, or smaller. These droplet sizes can conveniently be achieved by techniques such as microfluidisation.

In some embodiments, and particularly where the β-glucan is of the laminarin type, a composition is preferably not adjuvanted with complete Freund's adjuvant or with a wild-type cholera toxin.

Antigens and adjuvants in a composition will typically be in admixture.

Where an aluminium salt is used as an adjuvant for a conjugate then it is preferred that at least 50% by mass of the conjugate in a composition is adsorbed to the salt e.g. >60%, >70%, >80%, >90%, >95%, >98%, >99% or 100%. Adsorption of >99% can readily be achieved with a hydroxide salt.

Compositions may include two or more of said adjuvants. As shown in the examples, such combinations can improve the immune response elicited by glucan conjugates. Individual adjuvants may preferentially induce either a Th1 response or a Th2 response, and useful combinations of adjuvants can include both a Th2 adjuvant (e.g. an oil-in-water emulsion or an aluminium salt) and a Th1 adjuvant (e.g. 3dMPL, a saponin, or an immunostimulatory oligonucleotide). For example, compositions may advantageously comprise: both an aluminium salt and an immunostimulatory oligodeoxynucleotide; both an aluminium salt and a compound of formula I, II or III; both an oil-in-water emulsion and a compound of formula I, II or III; both an oil-in-water emulsion and an immunostimulatory oligodeoxynucleotide; both an aluminium salt and an α-glycosylceramide; both an oil-in-water emulsion and an α-glycosylceramide; both an oil-in-water emulsion and 3dMPL; both an oil-in-water emulsion and a saponin; etc.

### Pharmaceutical compositions

The invention provides a pharmaceutical composition comprising (a) an immunogenic composition of the invention, (b) an adjuvant, as described above and (c) a pharmaceutically acceptable carrier. A thorough discussion of such carriers is available in reference 177.

Microbial infections affect various areas of the body and so the compositions of the invention may be prepared in various forms. For example, the compositions may be prepared as injectables, either as liquid solutions or suspensions. Solid forms suitable for solution in, or suspension in, liquid vehicles prior to injection can also be prepared. The composition may be prepared for topical administration *e.g.* as an ointment, cream or powder. The composition be prepared for oral administration *e.g.* as a tablet or capsule, or as a syrup (optionally flavoured). The composition may be prepared for pulmonary administration *e.g.* as an inhaler, using a fine powder or a spray. The composition may be prepared as a suppository or pessary. The composition may be prepared for nasal, aural or ocular administration e.g. as drops, as a spray, or as a powder [*e.g.* 178].

The pharmaceutical composition is preferably sterile. It is preferably pyrogen-free.

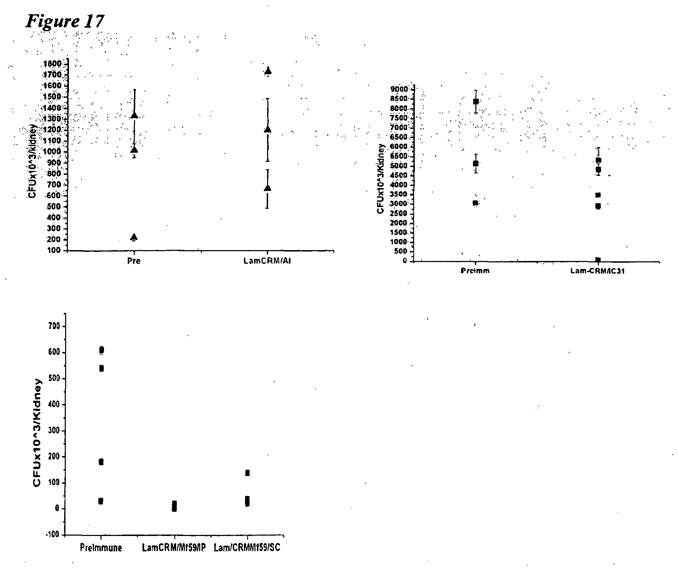

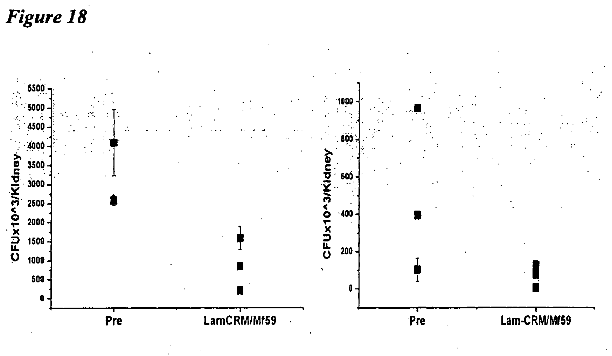

It is preferably buffered *e.g.* at between pH 6 and pH 8, generally around pH 7. The composition may be aqueous, or it may be lyophilised. The inventors have found that liquid formulations of the pharmaceutical compositions of the invention may be unstable (Figures 17 and 18). Accordingly, lyophilised formulations may be preferred. On the other hand, the inventors have also found that oil-in-water emulsion adjuvants, and in particular MF59, can improve the stability of liquid formulations of the pharmaceutical compositions (Figures 17 and 18). Accordingly, when the pharmaceutical compositions are prepared as liquid formulations, it may be preferred for the adjuvant to include an oil-in-water emulsion.

The invention also provides a delivery device containing a pharmaceutical composition of the invention. The device may be, for example, a syringe or an inhaler.

Pharmaceutical compositions of the invention are immunogenic compositions, in that they comprise an immunologically effective amount of a glucan immunogen. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (e.g. non-human primate, primate, *etc.*), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials.

Once formulated, the compositions of the invention can be administered directly to the subject. The subjects to be treated can be animals; in particular, human subjects can be treated.

Immunogenic compositions of the invention may be used therapeutically (*i.e.* to treat an existing infection) or prophylactically (*i.e.* to prevent future infection). Therapeutic immunisation is particularly useful for treating *Candida* infection in immunocompromised subjects.

### Medical treatments and uses

The invention also provides an immunogenic composition of the invention, for use in medicine e.g. for use in raising an antibody response in a mammal. The invention also provides a method for raising an immune response in a mammal, comprising administering an immunogenic composition or pharmaceutical composition of the invention to the mammal.

The invention also provides the use of (i) an immunogenic composition of the invention and (ii) an adjuvant, in the manufacture of a medicament for preventing or treating a microbial infection in a mammal.

The immune response raised by these methods and uses will generally include an antibody response, preferably a protective antibody response. Methods for assessing antibody responses after saccharide immunisation are well known in the art. The antibody response is preferably an IgA or IgG response. The immune response may be prophylactic and/or therapeutic. The mammal is preferably a human. Because glucans (and β-glucans in particular) are an essential and principal polysaccharide constituent of almost all pathogenic fungi, particularly those involved in infections in immunocompromised subjects, and also in bacterial pathogens and protozoa, anti-glucan immunity may have efficacy against a broad range of pathogens and diseases. For example, anti-glucan serum raised after immunisation with *S.cerevisiae* is cross-reactive with *C.albicans.* Broad spectrum immunity is particularly useful because, for these human infectious fungal agents, chemotherapy is scanty, antifungal drug resistance is emerging and the need for preventative and therapeutic vaccines is increasingly recognized.

The uses and methods of the invention are particularly useful for treating/protecting against infections of: *Candida* species, such as *Calbicans; Cryptococcus* species, such as *C.neoformans; Enterococcus* species, such as *E.faecalis; Streptococcus* species, such as *S.pneumoniae, S.mutans, S.agalactiae* and *S.pyogenes; Leishmania* species, such as *L.major*; *Acanthamoeba* species, such as *A.castellani*; *Aspergillus* species, such as *A.fumigatus* and *A.flavus*; *Pneumocystis* species, such as *P.carinii*; *Mycobacterium* species, such as *M.tuberculosis; Pseudomonas* species, such as *P.aeruginosa*; *Staphylococcus* species, such as *S.aureus; Salmonella* species, such as *S.typhimurium*; *Coccidioides* species such as *C.immitis; Trichophyton* species such as *T.verrucosum*; *Blastomyces* species such as *B.dermatidis*; *Histoplasma* species such as *H.capsulatum*; *Paracoccidioides* species such as *P.brasiliensis*; *Pythium* species such as *P.insidiosum*; and *Escherichia* species, such as *E.coli.*

The uses and methods are particularly useful for preventing/treating diseases including, but not limited to: candidiasis (including hepatosplenic candidiasis, invasive candidiasis, chronic mucocutaneous candidiasis and disseminated candidiasis); candidemia; aspergillosis, cryptococcosis, dermatomycoses, sporothrychosis and other subcutaneous mycoses, blastomycosis, histoplasmosis, coccidiomycosis, paracoccidiomycosis, pneumocystosis, thrush, tuberculosis, mycobacteriosis, respiratory infections, scarlet fever, pneumonia, impetigo, rheumatic fever, sepsis, septicaemia, cutaneous and visceral leishmaniasis, corneal acanthamoebiasis, cystic fibrosis, typhoid fever, gastroenteritis and hemolytic-uremic syndrome. *Anti-C.albicans* activity is particularly useful for treating infections in AIDS patients.

Efficacy of immunisation can be tested by monitoring immune responses against β-glucan (*e.g.* anti-β-glucan antibodies) after administration of the composition. Efficacy of therapeutic treatment can be tested by monitoring microbial infection after administration of the composition of the invention.

Compositions of the invention will generally be administered directly to a patient. Direct delivery may be accomplished by parenteral injection (*e.g.* subcutaneously, intraperitoneally, intravenously, intramuscularly, or to the interstitial space of a tissue), or by rectal, oral, vaginal, topical, transdermal, intradermal, ocular, nasal, aural, or pulmonary administration. Injection or intranasal administration is preferred. Subcutaneous or intrapertioneal administration are particularly preferred. Intramuscular administration is also preferred.

The invention may be used to elicit systemic and/or mucosal immunity.

Vaccines prepared according to the invention may be used to treat both children and adults. Thus a subject may be less than 1 year old, 1-5 years old, 5-15 years old, 15-55 years old, or at least 55 years old. Preferred subjects for receiving the vaccines are the elderly (*e.g.* ≥50 years old, ≥60 years old, and preferably ≥65 years), or the young (*e.g.* ≤5 years old). The vaccines are not suitable solely for these groups, however, and may be used more generally in a population.

Treatment can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Administration of more than one dose (typically two doses) is particularly useful in immunologically naïve patients. Multiple doses will typically be administered at least 1 week apart (*e.g.* about 2 weeks, about 3 weeks, about 4 weeks, about 6 weeks, about 8 weeks, about 10 weeks, about 12 weeks, about 16 weeks, *etc.*). Where a multiple dose schedule is used then at least one dose will include an adjuvanted glucan, but another dose (typically a later dose) may include an unadjuvanted glucan. Similarly, at least one dose may include a conjugated glucan, but another dose (typically later) may include an unconjugated glucan.

Conjugates of the invention may be combined with non-glucan antigens into a single composition for simultaneous immunisation against multiple pathogens. As an alternative to making a combined vaccine, conjugates may be administered to patients at substantially the same time as (e.g. during the same medical consultation or visit to a healthcare professional or vaccination centre) other vaccines. Antigens for use in these combination vaccines or for concomitant administration include, for instance, immunogens from *Streptococcus agalactiae, Staphylococcus aureus and*/*or Pseudomonas aeuruginosa.*

Compositions of the invention may be used in conjunction with anti-fungals, particularly where a patient is already infected. The anti-fungal offers an immediate therapeutic effect whereas the immunogenic composition offers a longer-lasting effect. Suitable anti-fungals include, but are not limited to, azoles (*e.g.* fluconazole, itraconazole), polyenes (*e.g.* amphotericin B), flucytosine, and squalene epoxidase inhibitors (*e.g.* terbinafine) [see also ref. 179]. The anti-fungal and the immunogenic composition may be administered separately or in combination. When administered separately, they will typically be administered within 7 days of each other. After the first administration of an immunogenic composition, the anti-fungal may be administered more than once.

### Definitions

The term "comprising" encompasses "including" as well as "consisting" *e.g.* a composition "comprising" X may consist exclusively of X or may include something additional *e.g.* X + Y.

The word "substantially" does not exclude "completely" *e.g.* a composition which is "substantially free" from Y may be completely free from Y. Where necessary, the word "substantially" may be omitted from the definition of the invention.

The term "about" in relation to a numerical value x means, for example, *x*±10%.

Unless specifically stated, a process comprising a step of mixing two or more components does not require any specific order of mixing. Thus components can be mixed in any order. Where there are three components then two components can be combined with each other, and then the combination may be combined with the third component, *etc.*

Where animal (and particularly bovine) materials are used in the culture of cells, they should be obtained from sources that are free from transmissible spongiform encaphalopathies (TSEs), and in particular free from bovine spongiform encephalopathy (BSE). Overall, it is preferred to culture cells in the total absence of animal-derived materials.

Where a compound is administered to the body as part of a composition then that compound may alternatively be replaced by a suitable prodrug.

### MODES FOR CARRYING OUT THE INVENTION

### Curdlan conjugation (1)

Curdlan with a starting MW of >100kDa was treated by acid hydrolysis using HCl (0.5M) in DMSO for 10 minutes at 85°C. The hydrolysate had a DP around 25 units.

Hydrolysed material was neutralised with sodium phosphate buffer (400mM, pH 6.8) and diluted with water to give a 10:1 dilution of the starting material. The final concentration was 1 mg/ml. After dilution, some precipitation was detectable. The precipitates are probably high MW saccharide.

Ammonium acetate was added and then sodium cyanoborohydride. After adjusting the pH to 7.0 the mixture was incubated at 37°C for 3-5 days. This treatment introduced a primary amino group at the reducing terminus of the curdlan fragments. The amino-saccharides were then purified by ultrafiltration with a 3 kDa cut-off membrane. Amino groups were estimated by the Habeeb method.

Dried amino-oligosaccharide was solubilised in distilled water at a 40mM amino group concentration, then 9 volumes of DMSO were added followed by triethyl-amine at a final concentration of 200mM. To the resulting solution, adipic acid N-hydroxysuccinimido diester was added for a final concentration of 480 mM. Ester groups generated in this way were estimated by analysis of released N-hydroxysuccinimido groups.

Dried activated oligosaccharide was added to CRM₁₉₇ in 10mM phosphate buffer pH 7.0. The reaction was maintained under stirring at room temperature overnight. The final material had a ratio of about 50:1 in term of mol of N-hydroxysuccinimido ester per mol of protein..

The conjugate was then purified by ultrafiltration with a 30 kDa cut-off membrane. The conjugate was characterized by SDS-Page, SEC-HPLC and NMR. Also, the saccharide (total and un-conjugated saccharide) and protein content were estimated.

Similar work was carried out using tetanus toxoid as the carrier instead of CRM197.

For five prepared lots of conjugates, the saccharide:protein ratios were as follows (excess carrier):

| **Lot** | 1 | 2 | 3 | 4 | 5 |
|---|---|---|---|---|---|
| **Carrier** | CRM197 | CRM197 | CRM197 | CRM197 | Tt |
| **Ratio** | 0.46:1 | 0.25:1 | 0.45:1 | 0.35:1 | 0.29:1 |

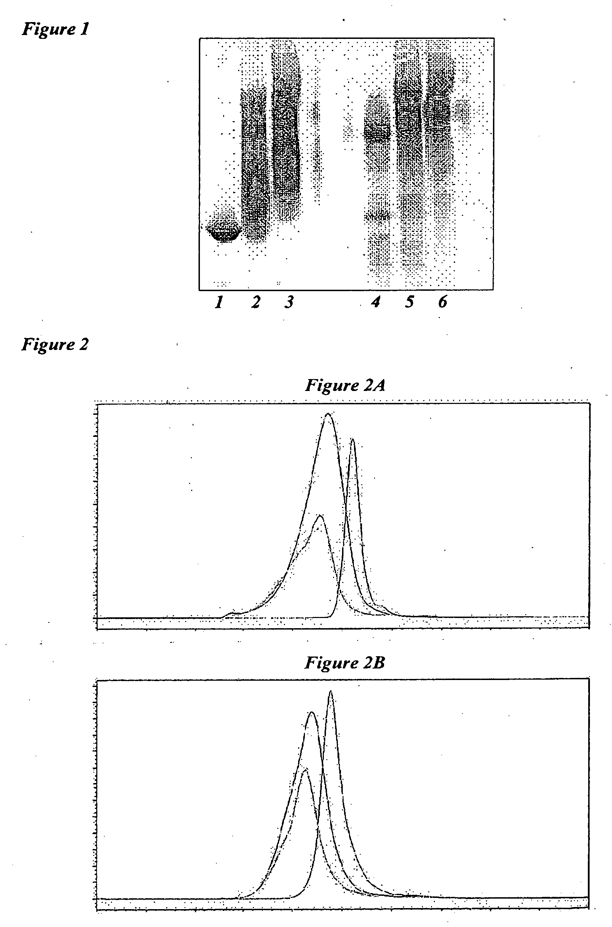

Figure 1 shows SDS-PAGE of example conjugates, and Figure 2 shows their SEC-HPLC profiles.

### Laminarin conjugation and adsorption (2)

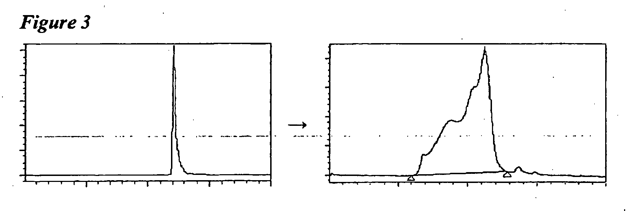

Laminarin conjugates were prepared as disclosed in references 2 and 3, using CRM197 carrier. One such conjugate had a saccharide:protein mass ratio of 0.4:1. After purification, 0.7% of free glucan (unconjugated) remained present. Figure 3 shows HPLC-SEC analysis of the carrier protein prior to conjugation, and of the final conjugate.

Conjugates were prepared in the same way, but with tetanus toxoid as the carrier instead of CRM197.

For five other lots of conjugates, the saccharide:protein ratios were as follows (excess carrier):

| **Lot** | 1 | 2 | 3 | 4 | 5 |
|---|---|---|---|---|---|
| **Carrier** | CRM197 | CRM197 | CRM197 | CRM197 | Tt |
| **Ratio** | 0.55:1 | 0.37:1 | 0.43:1 | 0.27:1 | 0.16:1 |

A CRM197 conjugate ('CRM-Lam') was combined with an aluminium hydroxide salt, at a fmal adjuvant concentration of 2mg/ml. Sodium chloride was included at 9mg/ml, and sodium phosphate at 10mM. The final composition had pH 7.0 and included 46.6µg/ml glucan, thus giving 7µg/dose at a 150µl dose volume (suitable for mouse studies).

The final composition was analysed by SDS-PAGE. In addition, to determine the degree of adsorption, it was centrifuged and its supernatant was analysed in parallel. TCA precipitation was also used, and the supernatant was again analysed. For comparison, unadsorbed material was also analysed, as was unconjugated CRM197 in both free and adsorbed form. In the presence of the adjuvant, no bands were detected in the supernatants of either CRM-Lam and or unconjugated CRM.

Adsorption was also assessed by analysing the supernatants by HPLC-SEC. Peaks at 214nm were measured, and the degree of adsorption for CRM-Lam was calculated as 99.7%.

### Conjugation (3)

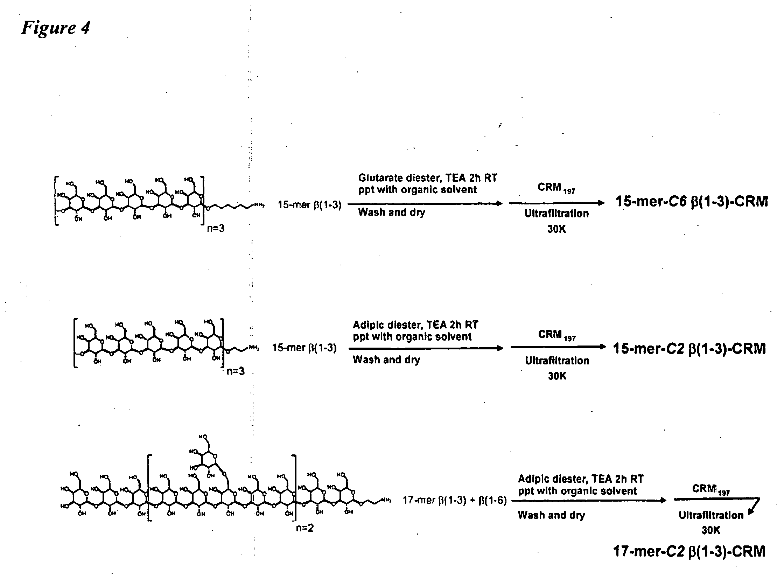

Synthetic curdlan (15-mer) and laminarin (17-mer) conjugates were prepared according to the method described in Figure 4. Briefly, the indicated synthetic oligosaccharides were solubilised in distilled water at a concentration of 40mM amino groups. Nine volumes of DMSO were then added, followed by triethylamine to a final concentration of 200mM. For the 15-mer-C6 β(1-3)-CRM conjugate, glutarate N-hydroxysuccinimido diester was added to a final concentration of 240 mM. For the 15-mer-C6 β(1-3)-CRM and 17-mer-C6 β(1-3)-CRM conjugates, adipic acid N-hydroxysuccinimido diester was added to a final concentration of 480 mM. The activated oligosaccharides were then purified by precipitation with 80% v/v dioxane. The number of ester groups generated in each reaction was estimated by measuring the amount of released N hydroxy-succinimido groups. Dried, activated oligosaccharides were then added to a 30 mg/mL CRM197 solution in 10mM phosphate buffer at pH 7.2. The reaction was maintained under stirring at room temperature overnight. The final materials had a ratio of about 50:1 in terms of moles of N-hydroxysuccinimido ester per mole of protein.

The conjugates were then characterized by SDS-Page and SEC-HPLC. The saccharide and protein contents were estimated as follows:

| **Sample** | **Conc sacc (mg/mL)** | **Conc prot (mg/mL)** | **Sacc/prot (%w/w)** | **Sacc/prot (mol/mol)** |
|---|---|---|---|---|
| 15-mer-C6 β(1-3)-CRM | 923.1 | 1695.5 | 54.4 | 11.7 |
| 15-mer-C2 β(1-3)-CRM | 651.7 | 2071.0 | 31.5 | 6.8 |
| 17-mer-C2 β(1-3)-CRM | 2113.7 | 4096.0 | 51.7 | 9.8 |

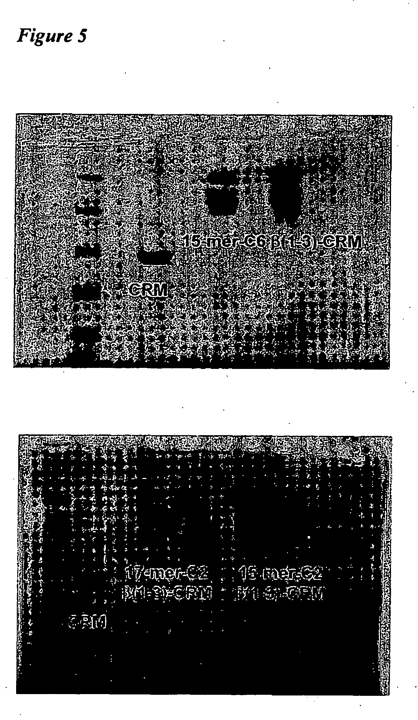

Figure 5 illustrates an SDS-PAGE analysis of these conjugates on a 7% tris-acetate gel (20 µg loaded per well).

### Laminarin conjugation (4)

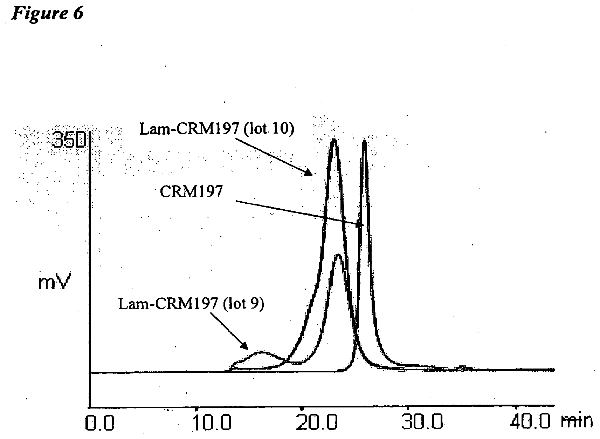

Further lots of laminarin conjugates were prepared as disclosed in references 2 and 3, except that the concentration of the phosphate in the buffer was either a) 10 mM (as per references 2 and 3, hereinafter "lot 9"); b) 25 mM; c) 50 mM or d) 100 mM ("lot 10"). The conjugates were then characterized by SEC-HPLC. Greater aggregation was observed in lot 9 than in the other lots, with no aggregation being detectable in lot 10 (Figure 6).Immunogenicity study (1)

To test immunogenicity, laminarin conjugates prepared as described in *Laminarin conjugation and adsorption* (2) were combined with various individual and combined adjuvants and tested in mice. CD2F1 mice, 4-6 weeks old, were tested in 12 groups of 10. The conjugates were used at a saccharide dose of 7µg in a dosage volume of 150µl, administered days 1, 7 and 21. Blood samples were taken on days 0, 21 and 35 for assessing anti-GGZym antibody levels by ELISA [2,3].

Groups 2 & 3 were primed (day 1) using the conjugate in combination with Complete Freund's adjuvant (CFA), solely for comparison purposes. Group 1 received CFA-adjuvanted laminarin and CRM197, but these were not conjugated. Group 4 was primed with unconjugated laminarin plus CFA. Group 5 received CFA alone. These five groups then received a mixture of laminarin and CRM197 with out adjuvant (group 1), unadjuvanted conjugate (group 2), unadjuvanted laminarin (groups 3 & 4) or PBS (group 5) at days 7 and 21.

Groups 7-8 and 9-12 received three identical doses of conjugated laminarin with the following adjuvants: (a) an aluminium hydroxide adjuvant; (b) the MF59 oil-in-water emulsion adjuvant; (c) a CpG oligodeoxynucleotide, CpG1826; (e) a combination of (a) and (c); (f) a combination of (b) and (c). Groups 6 and 9 received the same as groups 7 and 8, respectively, but the laminarin and CRM197 were not conjugated.

Anti-glucan antibodies (GMT) and the number of responding mice (%) at day 35 are reported in Table 1.

**Table 1**

| **Group** | **Day 1** | **Days 7 & 21** | **GMT** | **% responders** |
|---|---|---|---|---|
| 1 | Lam + CRM + CFA | Lam + CRM | 5 | 20 |
| 2 | Lam-CRM + CFA | Lam-CRM | 276 | 80 |
| 3 | Lam-CRM + CFA | Lam | 21 | 40 |
| 4 | Lam + CFA | Lam | 3 | 10 |
| 5 | CFA | PBS | 2 | 0 |
| 6 | Lam + CRM + alum | | 2 | 0 |
| 7 | Lam-CRM + alum | | 1034 | 90 |
| 8 | Lam-CRM + alum + CpG | | 3700 | 100 |
| 9 | Lam + CRM + MF59 | | 2 | 0 |
| 10 | Lam-CRM + MF59 | | 731 | 90 |
| 11 | Lam-CRM + MF59 + CpG | | 2616 | 100 |
| 12 | Lam-CRM + CpG | | 670 | 90 |
| Lam-CRM = laminarin conjugated to CRM197 | | | | |
| Lam + CRM = combination of laminarin and CRM197, no conjugation | | | | |
| Lam = unconjugated laminarin, no CRM197 | | | | |
| CFA = complete Freund's adjuvant | | | | |
| PBS = phosphate-buffered saline | | | | |

The results show that an optimum immune response requires both conjugation and the presence of at least one adjuvant. Combinations of Th1 and Th2 adjuvants gave good results.

The IgG responses of mice in groups 2 and 11 were studied in more detail. In all three cases IgG subclasses 1 and 3 were present, but subclasses 2a and 2b were absent.

### Immunogenicity study (2)

In further work, both curdlan and laminarin conjugates prepared as described in *Curdlan conjugation (1)* and *Laminarin conjugation and adsorption (2)* respectively were administered to mice. More than one lot of curdlan conjugates was tested.

Experiments were essentially the same as in the first study, but conjugates were used at a saccharide dose of 5µg. Conjugates were administered either without adjuvant or with the following adjuvants: (a) an aluminium hydroxide adjuvant; (b) the MF59 oil-in-water emulsion adjuvant; (c) a combination of (a) with 10µg of a CpG oligodeoxynucleotide, CpG1826; (e) a combination of (b) with a CpG oligodeoxynucleotide.

Anti-glucan antibodies (GMT) and the number of responding mice (%) at day 35 are reported in Table 2.

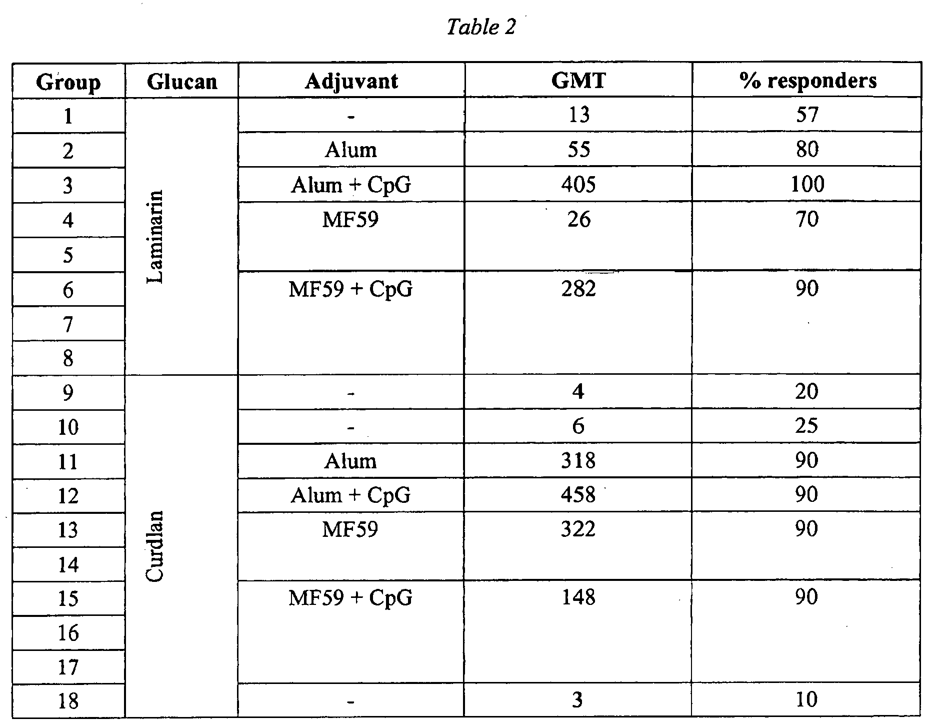

Although individual adjuvants were able to increase both the GMT and proportion of responders, for both laminarin and curdlan, combinations of adjuvants were particularly effective. Combinations of Th1 and Th2 adjuvants gave good results.

IgG responses in all groups were, as seen above, primarily in subclasses 1 and 3.

### Immunogenicity study (3)

In further experiments, laminarin and curdlan conjugates prepared as described in *Laminarin conjugation and adsorption (2)* and *Curdlan conjugation (1)* respectively were also adjuvanted with α-galactosylceramide (100ng) or LT-K63 (2µg), either alone or in combination with other adjuvants. The CpG adjuvant was also tested at three different doses (0.5µg, 5µg and 10µg). Details were as in the previous immunogenicity study, but with 8 mice per group. Results are in Table 3.

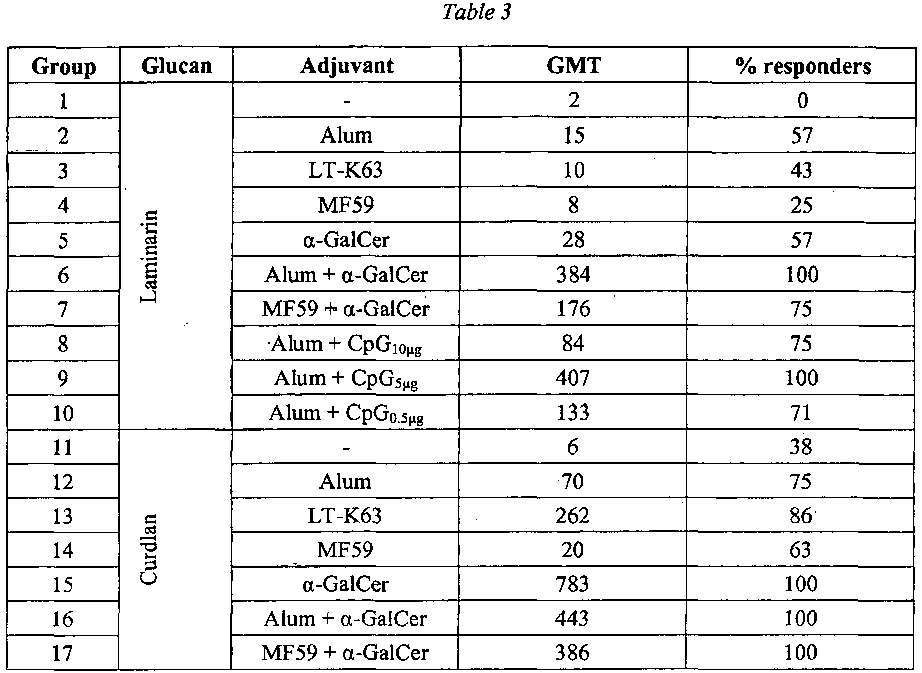

Again, adjuvant combinations gave the best results, except that α-GalCer was useful on its own for the curdlan conjugate. The best results from the CpG adjuvant were achieved at the middle dose.

### Immunogenicity study (4)

Mice, in groups of 16, were immunized intraperitoneally (IP) three times with laminarin conjugated to CRM197 (Lam-CRM, prepared as described in *Laminarin conjugation and adsorption* (2)) in combination with different adjuvants. The conjugates were used at a saccharide dose of 5 µg in a dosage volume of 150 µl, administered at days 1, 14 and 28. Blood samples were taken on days 0, 28 and 42 for assessing anti-GGZym antibody levels by ELISA. Anti-laminarin antibody levels were also measured by substituting laminarin for GG-Zym in the ELISA, as described in reference 3.

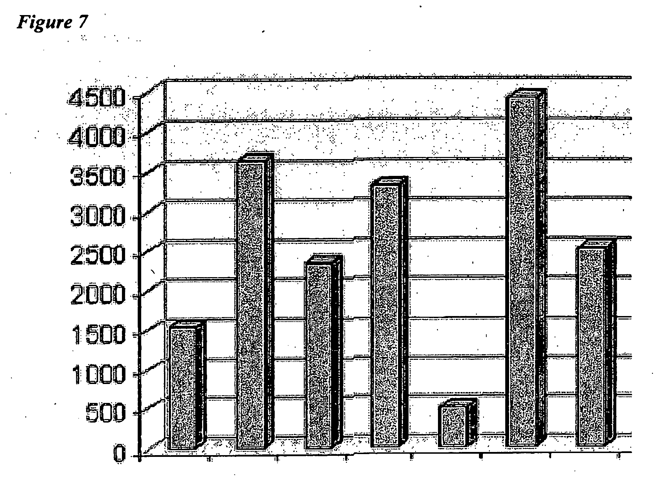

IgG GMTs against *Candida* cell wall glucan at day 35 are shown in Figure 7 (anti-GGZym antibody levels) and Table 4 (anti-GGZym and anti-laminarin antibody levels).

**Table 4**

| **Group** | **Mice #** | **Glucan** | **Adjuvant** | **Saccharide Dose** | **VPA** | **Route** | **GMT vs GGzym** | **GMT vs laminarin** |
|---|---|---|---|---|---|---|---|---|
| 1 | 16 | Laminarin | Alum | 5 µg | 150 µl | IP | 1534 | 4787 |
| 2 | 16 | Laminarin | Alum + CpG | 5 µg | 150 µl | IP | 3652 | 11277 |
| 3 | 16 | Laminarin | MF59 | 5 µg | 150 µl | IP | 2346 | 6760 |
| 4 | 16 | Laminarin | IC31 High | 5 pg | 150 µl | IP | 3318 | 15042 |
| 5 | 16 | Laminarin | IC31 Low | 5 µg | 150 µl | IP | 507 | 1138 |
| 6 | 16 | Laminarin | α-GalCer | 5 µg | 150 µl | IP | 4418 | 17762 |
| 7 | 16 | Laminarin | Alum/α-GalCer | 5 µg | 150 µl | IP | 2530 | 15238 |
| 8 | 16 | Laminarin | OMV nz | 5 µg | 150 µl | IP | 1465 | 1464 |
| 9 | 16 | Laminarin | Alum + OMV nz | 5 µg | 150 µl | IP | 2603 | 11465 |

The results show the immunogenicity of the glycoconjugate vaccine Lam-CRM in several different adjuvant formulations.

### Immunogenicity study (5)

In further work, laminarin or curdlan conjugated to either CRM197 or tetanus toxoid were combined with various individual and combined adjuvants and administered to mice by subcutaneous or intrapertioneal administration. The conjugates were prepared as described in *Laminarin conjugation and adsorption (2)* and *Curdlan conjugation (1)* respectively.

CD2F1 mice, 4-6 weeks old, were tested in 12 groups of 10. The conjugates were used at a saccharide dose of 5µg in a dosage volume of 150µl, administered days 1, 14 and 28 by subcutaneous or intrapertioneal administration. Blood samples were taken on days 0, 28 and 42 for assessing anti-GGZym antibody levels by ELISA.

Groups 1-3 received three identical doses of laminarin conjugated to CRM197 with the following adjuvants: (a) an aluminium hydroxide adjuvant (300µg); (b) a combination of (a) and a CpG oligodeoxynucleotide, CpG1826 (10µg); and (c) the MF59 oil-in-water emulsion adjuvant (75µl), respectively. Groups 4-6 were treated in the same way as groups 1-3 respectively, except that the glucan was curdlan instead of laminarin. Groups 7-9 were treated in the same way as groups 1-3 respectively, except that the laminarin was conjugated to tetanus toxoid instead of CRM197. Similarly, groups 10-12 were treated in the same way as groups 4-6 respectively, except that the curdlan was conjugated to tetanus toxoid instead of CRM197.

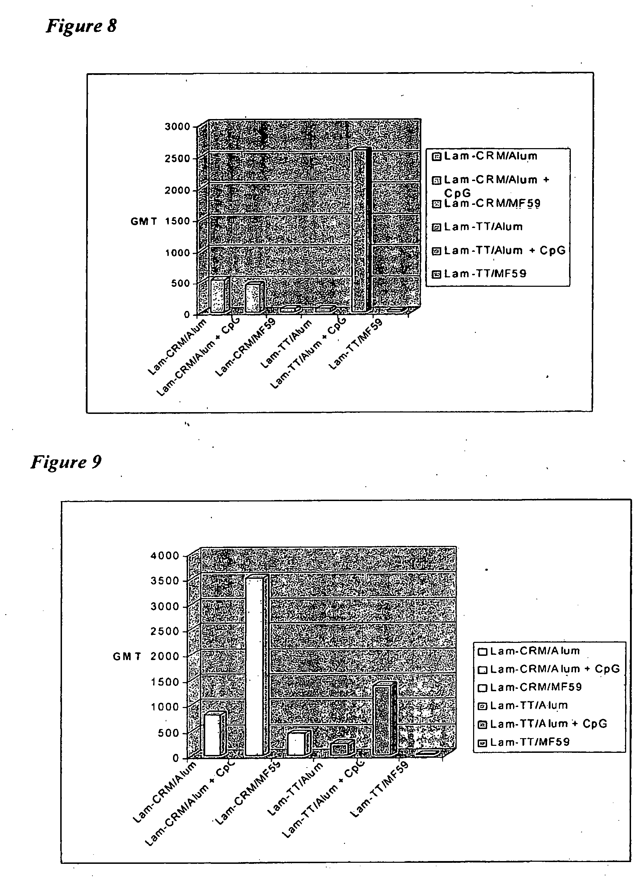

Anti-glucan antibodies (GMT) at day 42 after intrapertioneal administration of laminarin conjugates to the mice are shown in Figure 8. The corresponding results after subcutaneous administration are shown in Figure 9. The results show that a better response was generally seen when the conjugates were administered by subcutaneous administration. Moreover, better results were generally obtained using CRM197 as the carrier protein, particularly when the conjugates were administered by subcutaneous administration.

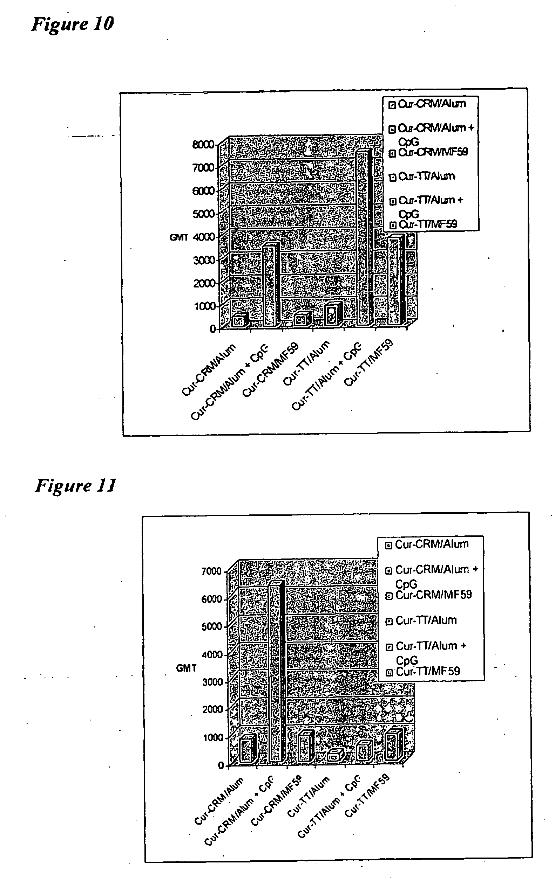

Similarly, anti-glucan antibodies (GMT) at day 42 after intrapertioneal administration of curdlan conjugates are shown in Figure 10. The corresponding results after subcutaneous administration are shown in Figure 11. When CRM197 was used as the carrier protein, a better response was seen when the conjugates were administered by subcutaneous administration.

### Immunogenicity study (6)

In another study, laminarin or curdlan conjugated to CRM197 were administered to mice using different doses of saccharide. The conjugates were prepared as described in *Laminarin conjugation and adsorption (2)* and *Curdlan conjugation (1)* respectively.

CD2F1 mice, 4-6 weeks old, were tested in 12 groups of 8. The conjugates were used at a saccharide doses of 10µg, 5µg, 1µg or 0.1µg in a dosage volume of 150µl, administered days 1, 14 and 28. Blood samples were taken on days 0, 28 and 42 for assessing anti-GGZym and anti-laminarin antibody levels by ELISA.

Group 1 received three identical doses of laminarin conjugated to CRM197 with no adjuvant and a saccharide dose of 5µg. Group 2 received three identical doses of laminarin conjugated to CRM197 with an aluminium hydroxide adjuvant (300 µg) and a saccharide dose of 5µg. A phosphate buffer had been used during the purification of the conjugate administered to this group. Groups 3-6 received three identical doses of laminarin conjugated to CRM197 with an aluminium hydroxide adjuvant (300 µg) and a saccharide dose of 10µg, 5µg, 1µg or 0.1µg, respectively. A histidine buffer had been used during the purification of the conjugates administered to these groups, as described in reference 180.

Groups 7-12 were treated in the same way as groups 1-6, except that the glucan was curdlan instead of laminarin.

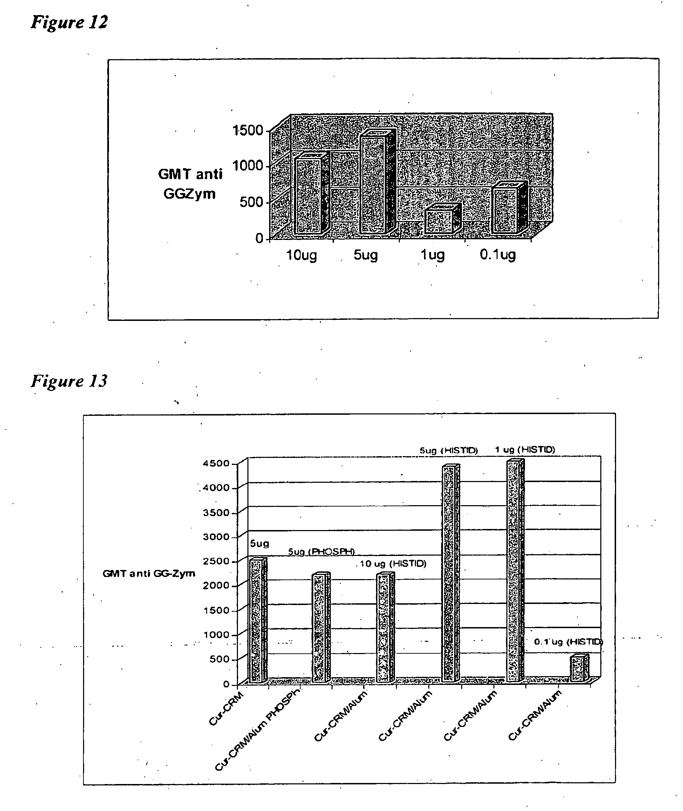

Anti-glucan antibodies (GMT) at day 42 after administration of laminarin conjugates at various saccharide doses are shown in Figure 12. The results show that a response was seen at all doses, with the best response being obtained with a saccharide dose of 5µg.

Anti-glucan antibodies (GMT) at day 42 after administration of the curdlan conjugates are shown in Figure 13. Once again, the results show that a response was seen at all doses of saccharide. The best responses were obtained with saccharide doses of 10µg and 5µg.

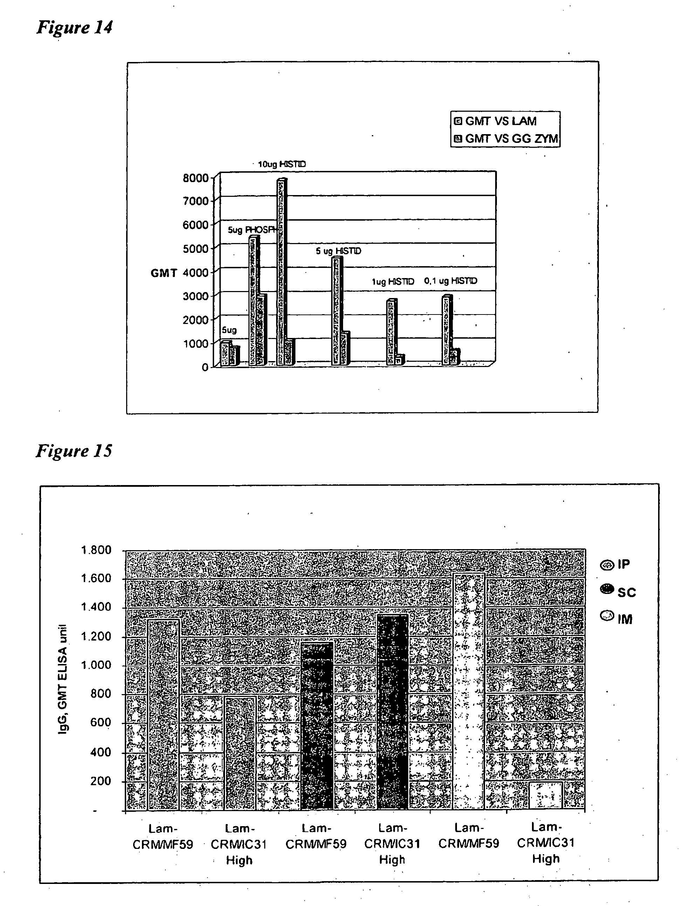

Anti-glucan antibodies (GMT) at day 42 after administration of the laminarin conjugates are shown in Figure 14. The results obtained using the anti-GGZym antibody ELISA are compared with those of the anti-laminarin antibody ELISA. Higher titres were observed using the anti-laminarin antibody ELISA.

### Immunogenicity study (7)

In further work, laminarin conjugated to CRM197 was combined with various individual adjuvants and administered to mice by intrapertioneal, by subcutaneous or intramuscular administration. The conjugate was prepared as described in *Laminarin conjugation and adsorption (2).*

CD2F1 mice, 4-6 weeks old, were tested in 6 groups of 16. The conjugates were used at a saccharide dose of 5µg in a dosage volume of 150µl, administered days 1, 14 and 28 by intrapertioneal, subcutaneous or intramuscular administration. Blood samples were taken on days 0, 28 and 42 for assessing anti-GGZym and anti-laminarin antibody levels by ELISA.

Groups 1-2 received three identical doses of laminarin conjugated to CRM197 by intrapertioneal administration with the following adjuvants: (a) the MF59 oil-in-water emulsion adjuvant (75µl); and (b) (4) IC31 at a high dose (49.5µl of a sample having over 1000 nmol/ml oligodeoxynucleotide and 40 nmol/ml peptide), respectively. Groups 3-4 were treated in the same way as groups 1-2 respectively, except that the conjugate was administered by subcutaneous administration. Groups 5-6 were treated in the same way as groups 1-2 respectively, except that the conjugate was administered by intramuscular administration.

Anti-glucan (anti-GGZym) antibodies (GMT) at day 42 after administration of laminarin conjugates are shown in Figure 15. The results show that better responses were generally seen when the conjugates were administered with the MF59 adjuvant. Moreover, the responses seen when the conjugates were administered with this adjuvant showed less dependence on the mode of administration: all of the modes of administration tested gave approximately the same response with this adjuvant. Similar results were obtained using the anti-laminarin antibody ELISA (Figure 16).

### Passive protection study

In another study, the ability of antibodies induced by laminarin conjugated to CRM197 combined with aluminium hydroxide, IC31 or MF59 adjuvants to inhibit the growth of C. *albicans in vivo* was tested. The conjugate was prepared as described in *Laminarin conjugation and adsorption (2).*

Separate pools of sera were obtained from mice used in *Immunogenicity study (1), Immunogenicity study (4)* and *Immunogenicity study (7)* above. A further pool of sera from pre-immune mice was obtained. Prior to use, the sera were inactivated by treatment at 56°C for 30 minutes.

CD2F1 mice, 4-6 weeks old, were tested in groups of 4-5. 0.5 ml of pooled sera was administered to each mouse by intrapertioneal administration. After two hours, each mouse was infected with 0.2ml of a culture of C. *albicans* by intravenous administration via the caudal vein such that each mouse received 5 x10⁵ CFU. After two days, each mouse was sacrificed and the left kidney removed. Each kidney was homogenised in the presence of 0.5ml PBS with 0.1% Triton X. Serial dilutions of the homogenates were plated onto Sabourad's agar and incubated for 48h at 28°C.

The accumulation of *C*. *albicans* in the kidneys of the mice treated with the pre- and post- immunization sera is shown in Figures 17 and 18. A lower accumulation could be observed in the groups of mice treated with the post-immunization sera from mice immunised with laminarin conjugated to CRM197 combined with at least the IC31 or MF59 adjuvants. Antibodies raised against laminarin conjugated to CRM197 combined with these adjuvants are therefore capable of inducing passive immunity. This effect was particularly clear for the antibodies raised against laminarin conjugated to CRM197 combined with the MF59 adjuvant.

### Laminarin purification

A 1mg/ml aqueous solution of a commercially-available laminarin extracted from *Laminaria digitata* (L-9634, Sigma) was analysed by UVNIS spectroscopy. The UV/VIS spectrum was obtained using a

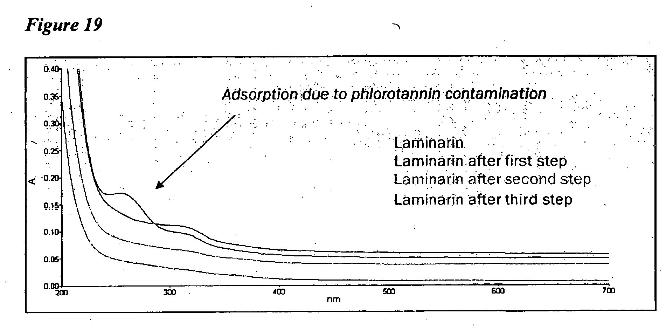

Perkin-Elmer LAMBDA™ 25 spectrophotometer at room temperature and pressure, using a quartz cell with a 1.00 cm path length. The same material was analysed after one, two or three steps of filtration using a depth filter (a Cuno™ 10 SP filter). The results are shown in Figure 19.

Phlorotannin contamination (as indicated by a UV absorbance peak at ∼270nm) was reduced after each step of filtration.

### Stability analysis

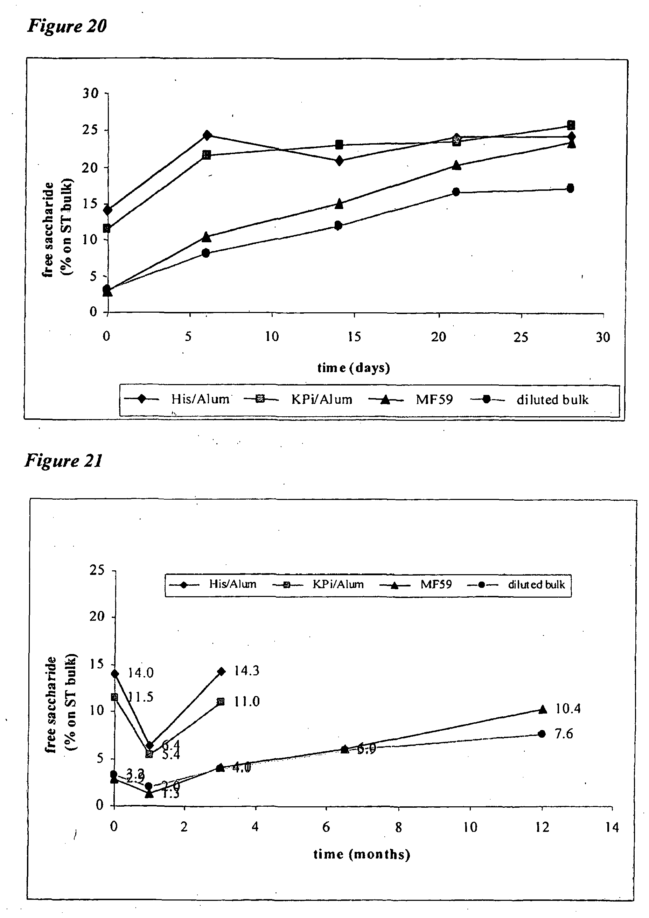

The stability of glucan conjugates formulated as liquids with various adjuvants was compared. The conjugate was prepared as described in *Laminarin conjugation and adsorption* (2). Samples were stored at 37°C for 4 weeks (Figure 20) and at 2-8 °C for six months (Figure 21). The release of glucan was monitored by measuring the % free saccharide at various time points. The determination of free saccharide is based on the separation of the free glucan from the conjugate by means of solid phase extraction (SPE) followed by quantitative determination of total and free glucan by means of high performance anion exchange chromatography-pulsed amperometric detection. The following formulations were tested:
Lam-CRM 20µg/mL in 10mM histidine buffer at pH7, 0.9% NaCl, 2mg/mL Al(OH)₃, 0.05% Tween 20;
Lam-CRM 20µg/mL in 10mM phosphate buffer at pH7, 0.9% NaCl, 2mg/mL Al(OH)₃,0.05% Tween 20;
Lam-CRM 20µg/mL in MF59; and
Lam-CRM 20µg/mL in 10mM phosphate buffer at pH7, 0.9% NaCl.

The results show that glucan conjugates combined with MF59 are more stable than glucan conjugates combined with aluminium hydroxide (in phosphate or histidine buffer).

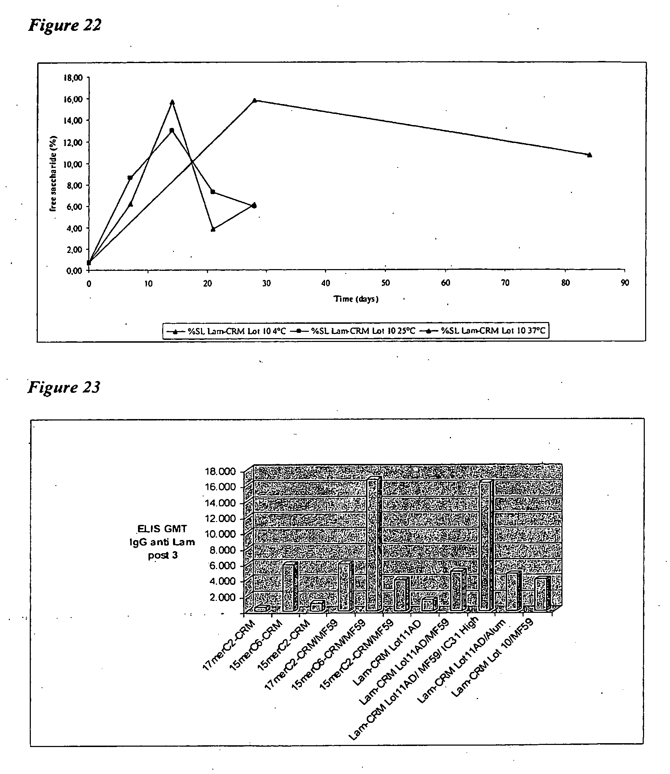

The stability of a lyophilised formulation of glucan conjugates was also measured. Samples were stored at 4, 25 or 37°C for up to 3 months (Figure 22). The following unit dose formulation was tested:
Lam-CRM 10µg/mL, sodium chloride 3.5 mg, sodium phosphate monobasic monohydrate 0.092mg, sodium phosphate dibasic dihydrate 0.48 mg, mannitol 7.3mg.

### Immunogenicity study (8)

In another study, conjugates prepared as described in *Conjugation (3)* and laminarin conjugated to CRM197 were combined with various individual and combined adjuvants and administered to mice by intrapertioneal administration. The laminarin conjugated to CRM197 was prepared as described in *Laminarin conjugation and adsorption (2),* except for an alternative lot of laminarin to CRM197 (lot 11AD) which was prepared without an amination step prior to conjugation.

CD2F1 mice, 4-6 weeks old, were tested in 11 groups of 16. The conjugates were used at a saccharide dose of 5µg in a dosage volume of 150µl, administered by intraperitoneal administration at days 1, 14 and 28. Blood samples were taken on days 0, 28 and 42 for assessing anti-laminarin antibody levels by ELISA.

Groups 1-3 received three identical doses of a) 17-mer-C2 β(1-3)-CRM conjugate; b) 15-mer-C6 β(1-3)-CRM conjugate; or c) 15-mer-C2 β(1-3)-CRM conjugate respectively, all with no adjuvant. Groups 4-6 received three identical doses of a) 17-mer-C2 β(1-3)-CRM conjugate; b) 15-mer-C6 β(1-3)-CRM conjugate; or c) 15-mer-C2 β(1-3)-CRM conjugate respectively, all with the MF59 oil-in-water emulsion adjuvant (75µl). Groups 7-8 received three identical doses of laminarin conjugated to CRM197 with a) no adjuvant; or b) the MF59 oil-in-water emulsion adjuvant (75µl) respectively. Groups 9-10 received three identical doses of laminarin conjugated to CRM197 with a) the MF59 oil-in-water emulsion adjuvant (75µl) combined with IC31 at a high dose (49.5µl of a sample having over 1000 nmol/ml oligodeoxynucleotide and 40 nmol/ml peptide); or b) an aluminium hydroxide adjuvant (300µg), respectively. Group 11 received three identical doses of a different preparation of laminarin conjugated to CRM197 with the MF59 oil-in-water emulsion adjuvant (75µl).

Anti-laminarin antibodies (GMT) at day 42 after administration of the conjugates are shown in Figure 23. The results show that the synthetic curdlan and laminarin conjugates have similar immunogenicity as the other conjugates. When an adjuvant is present, the immunogenicity may be improved by using a synthetic version of the relevant glucan (compare the response seen after administration of 17-mer-C2 β(1-3)-CRM/MF59 (bar 4) with the response seen after administration of laminarin conjugated to CRM197/MF59 (bars 7 and 11)). The immunogenicity of the synthetic glucans may be improved by using a longer spacer between the glucan and the carrier protein (compare the response seen after administration of 15-mer-C6 β(1-3)-CRM and 15-mer-C6 β(1-3)-CRM/MF59 (bars 2 and 5) with the response seen after administration of 15-mer-C2 β(1-3)-CRM and 15-mer-C2 β(1-3)-CRM/MF59 (bars 3 and 6)). In the absence of adjuvant, immunogenicity to the synthetic glucans may be improved by the absence of β-1,6-branching (compare the response seen after administration of 15-mer-C2 β(1-3)-CRM (bar 3) with the response seen after administration of 17-mer-C2 β(1-3)-CRM (bar 1). In contrast, in the presence of adjuvant, immunogenicity to the synthetic glucans may be improved by the presence of β-1,6-branching (compare the response seen after administration of 17-mer-C2 β(1-3)-CRM/MF59 (bar 4) with the response seen after administration of 15-mer-C2 β(1-3)-CRM/MF59 (bar 6). For the laminarin conjugated to CRM197, the omission of an amination step prior to conjugation did not prevent immunogenicity (compare bars 8 and 11).

### Active protection study (1)

In another study, the ability of mice receiving glucans conjugated to CRM197 combined with MF59 adjuvant to survive challenge with *C. albicans* was tested. The conjugates were prepared as described in *Laminarin conjugation (4)* (lots 9 and 10) and *Curdlan conjugation (1).*

Female, four-week old CD2F1 mice (Harlan) were immunized with three doses of laminarin or curdlan conjugated to CRM197, each dose consisting of 10µg polysaccharide in 0.2 ml of PBS:MF59 (1:1 v/v) per mouse.

The immunization schedule was:
- Day 0 - first dose by subcutaneous administration
- Day 14 - second dose by intraperitoneal administration
- Day 28 - third dose by intraperitoneal administration
- Day 35 - bleeding
- Day 40 - fungal challenge by intravenous administration of 5.0x10⁵ (after immunisation with the laminarin conjugate) or 2.5x10⁵ (after immunisation with the curdlan conjugate) *C.albicans* strain BP cells in 0.2 ml PBS per mouse.

Protection endpoints were measured in terms of mortality (median survival time (MST) and ratio of dead/total challenged mice).

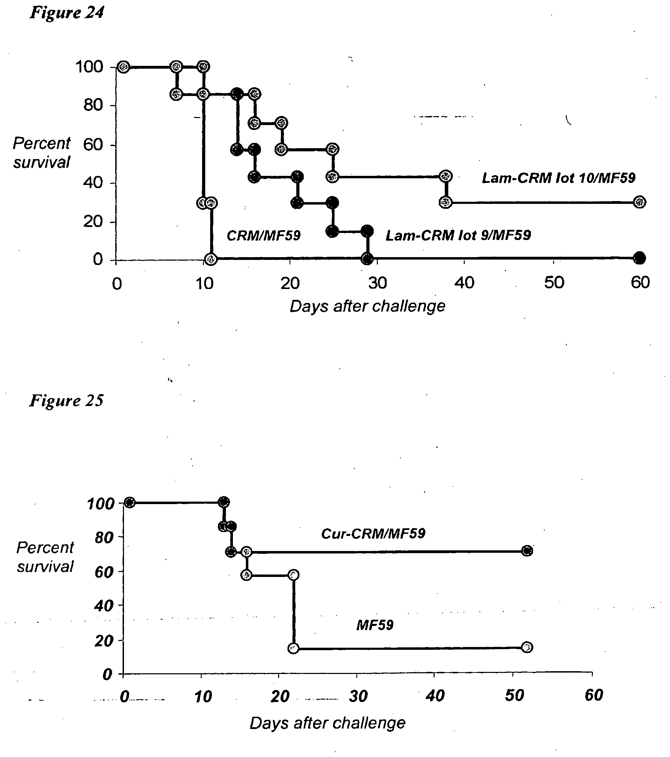

Figure 24 shows the survival rate of mice treated with laminarin conjugated to CRM197 combined with MF59 or CRM197 and MF59 alone prior to challenge with *C.albicans.* The longer survival of mice treated with the conjugate is also shown in terms of MST in Table 5.

**Table 5**

| **Vaccine** | **MST (days)** |
|---|---|
| CRM197/MF59 | 10 |
| Lam-CRM197 lot 9/MF59 | 16 |
| Lam-CRM197 lot 10/MF59 | 25 |

Survival was greater in mice receiving lot 10 than in mice receiving lot 9.

Figure 25 shows the survival rate of mice treated with curdlan conjugated to CRM197 combined with MF59 or MF59 alone prior to challenge with *C.albicans.* The longer survival of mice treated with the conjugate is also shown in terms of MST in Table 6.

**Table 6**

| **Vaccine** | **MST (days)** |
|---|---|
| MF59 | 16 |
| Cur-CRM197/MF59 | >52 |

Survival was greater in mice receiving curdlan conjugated to CRM197 than in mice receiving laminarin conjugated to CRM197.

### Active protection study (2)

In a similar study, the ability of mice receiving synthetic glucans conjugated to CRM197 combined with MF59 adjuvant to survive challenge with *C. albicans* was tested. The conjugates were prepared as described in *Conjugation (3)*. In this study, fungal challenge was by intravenous administration of 5.0x10⁵ cells.

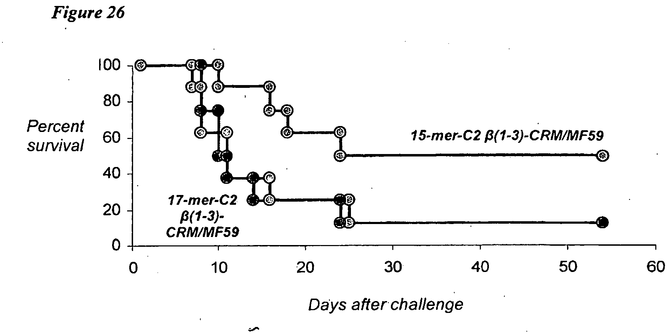

Figure 26 shows the survival rate of mice treated with 15-mer-C2 β(1-3)-CRM combined with MF59, 17-mer-C2 β(1-3)-CRM combined with MF59 or MF59 alone prior to challenge with *C.albicans.* The longer survival of mice treated with the 15-mer-C2 β(1-3)-CRM conjugate is also shown in terms of MST in Table 7.

**Table 7**

| **Vaccine** | **MST (days)** |
|---|---|
| MF59 | 11 |
| 17mer-C2-CRM197/MF59 | 10 |
| 15mer-C2-CRM197/MF59 | 24 |

Treatment with 15-mer-C2 β(1-3)-CRM resulted in increased survival, while treatment with 17-mer-C2 β(1-3)-CRM did not seem to have any effect. This result suggests that the epitope responsible for inducing a protective antibody response in glucan comprises at least five adjacent non-terminal residues linked to other residues only by β-1,3 linkages. Without wishing to be bound by theory, it is though that this effect may contribute to the greater protective antibody response seen in mice receiving curdlan conjugated to CRM197 than in mice receiving laminarin conjugated to CRM197 in *Active protection study (1).* The curdlan conjugated to CRM197 (wherein the glucan comprises β-1,3-linked residues only) may contain a greater proportion of protective epitopes than the laminarin conjugated to CRM197 (wherein the glucan comprises β-1,3-linked residues and β-1,6-linked residues).

It will be understood that the invention has been described by way of example only and modifications may be made whilst remaining within the scope of the invention, which is defined in the appended claims.

### REFERENCES

[1] Cassone & Torosantucci (2006) Expert Rev Vaccines 5:859-67.
[2] WO03/097091
[3] Torosantucci et al. (2005) JExp Med 202:597-606.
[7] Tokunaka et al. (1999) Carbohydr Res 316:161-172.
[8] Pang et al. (2005) Biosci Biotechnol Biochem 69:553-8.
[9] Read et al. (1996) Carbohydr Res. 281:187-201.
[10] Takeo and Tei (1986) Carbohydr Res. 145:293-306
[11] Tanaka et al. (2003) Tetrahedron Letters 44:3053-3057
[12] Ning et al. (2002) Tetrahedron Letters 43:5545-5549
[13] Geurtsen et al. (1999) Journal of Organic Chemistry 64 (21):7828-7835
[14] Wu et al. (2003) Carbohydr Res. 338:2203-12
[15] Nicolaou et al. (1997) J. Am. Chem. Soc. 119:449-450
[16] Yamada et al. (1999) Tetrahedron Letters 40:4581-4584
[17] Yamago et al. (2001) Org. Lett. 24:3867-3870
[18] Yuguo et al. (2004) Tetrahedron 60: 6345-6351
[19] Amaya et al. (2001) Tetrahedron Letters 42:9191-9194
[20] Mei et al. (2005) Carbohydr Res. 340:2345-2351
[21] Takeo et al. (1993) Carbohydr Res. 245:81-96
[22] Jamois et al. (2005) Glycobiology 15(4):393-407
[23] Lefeber et al. (2001) Chem. Eur. J. 7(20):4411-4421
[24] Huang et al. (2005) Carbohydr Res. 340:603-608
[25] US patent 5508191.
[26] MiKyoung et al. (2003) Biochemical Engineering Journal, 16:163-8.
[27] Barsanti et al. (2001) JAppl Phycol 13:59-65.
[28] Bardotti et al. (2008) Vaccine 26:2284-96
[30] Koivikko et al. (2007) Phytochem Anal. 18(4):326-32.
[31] Lindberg (1999) Vaccine 17 Suppl 2:S28-36
[32] Buttery & Moxon (2000) JR Coll Physicians Lond 34:163-8
[33] Ahmad & Chapnick (1999) Infect Dis Clin North Am 13:113-33, vii
[34] Goldblatt (1998) J. Med. Microbiol. 47:563-567
[35] EP-B-0 477 508
[36] US patent 5,306,492
[37] WO98/42721
[38] Dick et al. in Conjugate Vaccines (eds. Cruse et al.) Karger, Basel, 1989, Vol. 10, 48-114
[39] Hermanson Bioconjugate Techniques, Academic Press, San Diego CA (1996)
[40] Ramsay et al. (2001) Lancet 357(9251):195-6
[41] Lees et al. (1996) Vaccine 14:190-198.
[42] WO95/08348.
[43] WO98/42721
[44] US patent 4,761,283
[45] US patent 4,356,170
[46] US patent 4,882,317
[47] US patent 4,695,624
*[48]* Mol. Immunol., 1985, 22, 907-919
[49] EP-A-0208375
[50] Bethell G.S. et al., J. Biol. Chem., 1979, 254, 2572-4
[51] Hearn M.T.W., J. Chromatogr., 1981, 218, 509-18
[52] WO00/10599
[53] Gever et al., Med. Microbiol. Immunol, 165: 171-288 (1979).
[54] US patent 4,057,685.
[55] US patents 4,673,574; 4,761,283; 4,808,700.
[56] US patent 4,459,286.
[57] US patent 5,204,098
[58] US patent 4,965,338
[59] US patent 4,663,160.
[60] W02007/000343.
[61] Research Disclosure, 453077 (Jan 2002)
[62] EP-A-0372501.
[63] EP-A-0378881.
[64] EP-A-0427347.
[65] WO93/17712
[66] WO94/03208.
[67] WO98/58668.
[68] EP-A-0471177.
[69] WO91/01146
[70] Falugi et al. (2001) Eur J Immunol 31:3816-3824.
[71] Baraldo et al. (2004) Infect Immun 72(8):4884-7.
[72] EP-A-0594610.
[73] Ruan et al. (1990) J Immunol 145:3379-3384.
[74] WO00/56360.
[75] Kuo et al. (1995) Infect Immun 63:2706-13.
[76] Michon et al. (1998) Vaccine. 16:1732-41.
[77] WO02/091998.
[78] WO01/72337
[79] WO00/61761.
[80] WO00/33882
[81] WO99/42130
[82] WO96/40242
[83] Lei et al. (2000) Dev Biol (Basel) 103:259-264
[84] WO00/38711
[85] US patent 6355271.
[86] WO00/23105.
[87] US 5,057,540.
[88] WO96/33739.
[89] EP-A-0109942.
[90] WO96/11711.
[91] WO00/07621.
[92] Barr et al. (1998) Advanced Drug Delivery Reviews 32:247-271.
[93] Sjolanderet et al. (1998) Advanced Drug Delivery Reviews 32:321-338.
[94] Pizza et al. (2000) Int J Med Microbiol 290:455-461.
[95] Tebbey et al. (2000) Vaccine 18:2723-34.
[96] WO95/17211.
[97] WO98/42375.
[98] Singh et al] (2001) J Cont Release 70:267-276.
[99] WO99/27960.
[100] US 6,090,406
[101] US 5,916,588
[102] EP-A-0626169.
[103] Dyakonova et al. (2004) Int Immunopharmacol 4(13):1615-23.
[104] FR-2859633.
[105] Signorelli & Hadden (2003) Int Immunopharmacol 3(8):1177-86.
[106] W02004/064715.
[107] De Libero et al, Nature Reviews Immunology, 2005, 5: 485-496
[108] US patent 5,936,076.
[109] Oki et al, J. Clin. Investig., 113: 1631-1640
[110] US2005/0192248
[111] Yang et al, Angew. Chem. Int. Ed., 2004, 43: 3818-3822
[112] WO2005/102049
[113] Goff et al, J. Am. Chem., Soc., 2004, 126: 13602-13603
[114] WO03/105769
[115] Cooper (1995) Pharm Biotechnol 6:559-80.
[116] Kandimalla et al. (2003) Nucleic Acids Research 31:2393-2400.
[117] WO02/26757.
[118] WO99/62923.
[119] Krieg (2003) Nature Medicine 9:831-835.
[120] McCluskie et al. (2002) FEMS Immunology and Medical Microbiology 32:179-185.
[121] WO98/40100.
[122] US patent 6,207,646.
[123] US patent 6,239,116.
[124] US patent 6,429,199.
[125] Kandimalla et al. (2003) Biochemical Society Transactions 31 (part 3):654-658.
[126] Blackwell et al. (2003) J Immunol 170:4061-4068.
[127] Krieg (2002) Trends Immunol 23:64-65.
[128] WO01/95935.
[129] Kandimalla et al. (2003) BBRC 306:948-953.
[130] Bhagat et al. (2003) BBRC 300:853-861.
[131] WO03/035836.
[132] WO01/22972.
[133] Schellack et al. (2006) Vaccine 24:5461-72.
[134] yers et al. (1990) pages 145-156 of Cellular and molecular aspects of endotoxin reactions.
[135] Ulrich (2000) Chapter 16 (pages 273-282) of reference 171.
[136] Johnson et al. (1999) J Med Chem 42:4640-9.
[137] Baldrick et al. (2002) Regulatory Toxicol Pharmacol 35:398-413.
[138] WO 94/21292.
[139] US 4,680,338.
[140] US 4,988,815.
[141] WO92/15582.
[142] Stanley (2002) Clin Exp Dermatol 27:571-577.
[143] Wu et al. (2004) Antiviral Res. 64(2):79-83.
[144] Vasilakos et al. (2000) Cell Immunol. 204(1):64-74.
[145] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.
[146] Jones (2003) Curr Opin Investig Drugs 4:214-218.
[147] W02004/060308.
[148] W02004/064759.
[149] US 6,924,271.
[150] US2005/0070556.
[151] US 5,658,731.
[152] US patent 5,011,828.
[153] W02004/87153.
[154] US 6,605,617.
[155] WO02/18383.
[156] W02004/018455.
[157] WO03/082272.
[158] Johnson et al. (1999) Bioorg Med Chem Lett 9:2273-2278.
[159] Evans et al. (2003) Expert Rev Vaccines 2:219-229.
[160] Andrianov et al. (1998) Biomaterials 19:109-115.
[161] Payne et al. (1998) Adv Drug Delivery Review 31:185-196.
[162] WO03/011223.
[163] Meraldi et al. (2003) Vaccine 21:2485-2491.
[164] Pajak et al. (2003) Vaccine 21:836-842.
[165] Wong et al. (2003) J Clin Pharmacol 43(7):735-42.
[166] US2005/0215517.
[167] WO90/14837.
[168] Podda & Del Giudice (2003) Expert Rev Vaccines 2:197-203.
[169] Podda (2001) Vaccine 19: 2673-2680.
[170] Vaccine Design: The Subunit and Adjuvant Approach (eds. Powell & Newman) Plenum Press 1995 (ISBN 0-306-44867-X).
[171] Vaccine Adjuvants: Preparation Methods and Research Protocols (Volume 42 of Methods in Molecular Medicine series). ISBN: 1-59259-083-7. Ed. O'Hagan.
[172] Allison & Byars (1992) Res Immunol 143:519-25.
[173] Hariharan et al. (1995) Cancer Res 55:3486-9.
[174] WO95/11700.
[175] US patent 6,080,725.
[176] W02005/097181.
[177] Gennaro (2000) Remington: The Science and Practice of Pharmacy. 20th edition, ISBN: 0683306472.
[178] Almeida & Alpar (1996) J. Drug Targeting 3:455-467.
[179] Wills et al. (2000) Emerging Therapeutic Targets 4:1-32.
[180] WO03/009869

## Claims

1. An immunogenic composition comprising a glucan that has exclusively β-1,3-linkages, wherein said glucan is a single molecular species and is conjugated to a carrier protein, wherein the glucan comprises one or more sequences of at least five adjacent non-terminal residues linked to other residues only by β-1,3 linkages, and wherein the carrier protein is a bacterial toxin or toxoid, or a mutant thereof, preferably wherein the carrier protein is CRM197.

2. The composition of claim 1, wherein the glucan is conjugated to the carrier protein directly or via a linker.

3. The composition of any preceding claim, wherein the glucan has a molecular weight of less than 100 kDa and/or the glucan has 60 or fewer glucose monosaccharide units.

4. The composition of any preceding claim, wherein the glucan is a curdlan.

5. The composition of any preceding claim, including a pharmaceutically acceptable carrier.

6. The composition of any preceding claim, wherein the composition further comprises an adjuvant.

7. The composition of claim 6, wherein the adjuvant comprises one or more of: an aluminium salt; an oil-in-water emulsion; an immunostimulatory oligonucleotide; and/or an α-glycosylceramide, preferably wherein the adjuvant comprises an immunostimulatory oligonucleotide and a polycationic oligopeptide.

8. The composition of claim 7, wherein the adjuvant is MF59.

9. The composition of any preceding claim, wherein the glucan has a polydispersity (Mw/Mn) of 1.01 or less.

10. A composition of any preceding claim for use in a method for raising an immune response in a mammal, comprising administering to the mammal the composition.

## Patentansprüche

1. Immunogene Zusammensetzung, umfassend ein Glukan mit ausschließlich β-1,3-Verknüpfungen, wobei es sich bei dem Glukan um eine Einzelmolekülspezies handelt und das Glukan mit einem Trägerprotein konjugiert ist, wobei das Glukan eine oder mehrere Sequenzen von wenigstens fünf benachbarten nicht terminalen Resten, die mit anderen Resten nur über β-1,3-Verknüpfungen verknüpft sind, umfasst, und wobei es sich bei dem Trägerprotein um ein bakterielles Toxin oder Toxoid oder eine Mutante davon handelt, wobei es sich bei dem Trägerprotein vorzugsweise um CRM197 handelt.

2. Zusammensetzung nach Anspruch 1, wobei das Glukan mit dem Trägerprotein direkt oder über einen Linker konjugiert ist.

3. Zusammensetzung nach einem vorhergehenden Anspruch, wobei das Glukan ein Molekulargewicht von weniger als 100 kDa und/oder das Glukan 60 oder weniger Glucose-Monosaccharideinheiten aufweist.

4. Zusammensetzung nach einem vorhergehenden Anspruch, wobei es sich bei dem Glukan um ein Curdlan handelt.

5. Zusammensetzung nach einem vorhergehenden Anspruch, enthaltend einen pharmazeutisch unbedenklichen Trägerstoff.

6. Zusammensetzung nach einem vorhergehenden Anspruch, wobei die Zusammensetzung ferner ein Adjuvans umfasst.

7. Zusammensetzung nach Anspruch 6, wobei das Adjuvans eines oder mehrere der folgenden umfasst: ein Aluminiumsalz; eine Öl-in-Wasser-Emulsion; ein immunstimulatorisches Oligonukleotid; und/oder ein α-Glycosylceramid, wobei das Adjuvans vorzugsweise ein immunstimulatorisches Oligonukleotid und ein polykationisches Oligopeptid umfasst.

8. Zusammensetzung nach Anspruch 7, wobei es sich bei dem Adjuvans um MF59 handelt.

9. Zusammensetzung nach einem vorhergehenden Anspruch, wobei das Glukan eine Polydispersität (Mw/Mn) von 1,01 oder weniger aufweist.

10. Zusammensetzung nach einem vorhergehenden Anspruch zur Verwendung bei einem Verfahren zum Hervorrufen einer Immunantwort bei einem Säuger, wobei dem Säuger die Zusammensetzung verabreicht wird.

## Revendications

1. Composition immunogène comprenant un glucane possédant des liaisons exclusivement de type β-1, 3, dans laquelle ledit glucane est une espèce moléculaire unique et est conjugué à une protéine support, dans laquelle le glucane comprend une ou plusieurs séquences d'au moins cinq résidus non terminaux adjacents liés à d'autres résidus uniquement par des liaisons β-1, 3, et dans laquelle la protéine support est une toxine bactérienne ou un toxoïde bactérien, ou un mutant de ceux-ci, de préférence dans laquelle la protéine support est le CRM197.

2. Composition selon la revendication 1, dans laquelle le glucane est conjugué à la protéine support directement ou par l'intermédiaire d'un lieur.

3. Composition selon l'une quelconque des revendications précédentes, dans laquelle le glucane a un poids moléculaire inférieur à 100 kDa et/ou le glucane possède 60 unités monosaccharidiques de glucose ou moins.

4. Composition selon l'une quelconque des revendications précédentes, dans laquelle le glucane est un curdlane.

5. Composition selon l'une quelconque des revendications précédentes, comprenant un support pharmaceutiquement acceptable.

6. Composition selon l'une quelconque des revendications précédentes, dans laquelle la composition comprend en outre un adjuvant.

7. Composition selon la revendication 6, dans laquelle l'adjuvant comprend un ou plusieurs parmi : un sel d'aluminium ; une émulsion aqueuse ; un oligonucléotide immunostimulatoire ; et/ou un α-glycosylcéramide, de préférence dans laquelle l'adjuvant comprend un oligonucléotide immunostimulatoire et un oligopeptide polycationique.

8. Composition selon la revendication 7, dans laquelle l'adjuvant est le MF59.

9. Composition selon l'une quelconque des revendications précédentes, dans laquelle le glucane a une polydispersité (Mw/Mn) inférieure ou égale à 1,01.

10. Composition selon l'une quelconque des revendications précédentes destinée à être utilisée dans un procédé destiné à susciter une réponse immunitaire chez un mammifère, comprenant l'administration de la composition au mammifère.
